(19) Europäisches Patentamt
European Patent Office
Office européen des brevets

(11) **EP 1 622 629 B1**

(12) **EUROPEAN PATENT SPECIFICATION**

(45) Date of publication and mention
of the grant of the patent:
**10.07.2013 Bulletin 2013/28**

(51) Int Cl.:
*A61K 33/24* (2006.01)      *A61P 19/08* (2006.01)
*A61K 9/20* (2006.01)      *A61K 9/28* (2006.01)
*A61K 9/26* (2006.01)

(21) Application number: **04731307.7**

(22) Date of filing: **06.05.2004**

(86) International application number:
**PCT/DK2004/000326**

(87) International publication number:
**WO 2004/098617 (18.11.2004 Gazette 2004/47)**

(54) **Controlled-release composition containing a strontium salt**

Strontiumzubereitung mit kontrollierter Freisetzung

Composition a libération controlée contenant un sel de strontium

(84) Designated Contracting States:
**AT BE BG CH CY CZ DE DK EE ES FI FR GB GR
HU IE IT LI LU MC NL PL PT RO SE SI SK TR**

(30) Priority: **07.05.2003 DK 200300691
08.07.2003 DK 200301043
09.12.2003 DK 200301821
09.12.2003 US 528409 P**

(43) Date of publication of application:
**08.02.2006 Bulletin 2006/06**

(73) Proprietor: **Osteologix A/S
2100 Copenhagen (DK)**

(72) Inventors:
• **HANSEN, Christian
DK-2950 Vedbaek (DK)**
• **NILSSON, Henrik
DK-1053 Copenhagen K (DK)**
• **ANDERSEN, Jens, E., T.
DK-2950 Vedbaek (DK)**
• **CHRISTGAU, Stephan
DK-2820 Gentofte (DK)**

(74) Representative: **Hjelmencrantz, Anders et al
Chas. Hude A/S
H.C. Andersens Boulevard 33
1780 Copenhagen V (DK)**

(56) References cited:
EP-A- 0 381 445      EP-A- 0 404 558
WO-A-98/35657      WO-A-99/34772

WO-A1-03/028742      US-A- 5 856 356

• MOROHASHI T ET AL: "EFFECTS OF
STRONTIUM ON CALCIUM METABOLISM IN
RATS I. A DISTINCTION BETWEEN THE
PARMACOLOGICAL AND TOXIC DOSES"
JAPANESE JOURNAL OF PHARMACOLOGY,
THE JAPANESE PHARMACOLOGICAL SOCIETY,
KYOTO, JP, vol. 64, no. 3, 1994, pages 155-162,
XP008036785 ISSN: 0021-5198
• BRANDI M L: "New perspectives in the prevention
and treatment of glucocorticoid-induced
osteoporosis" CLINICAL AND EXPERIMENTAL
RHEUMATOLOGY 2000 ITALY, vol. 18, no. 5
SUPPL. 21, 2000, pages S74-S78, XP002314920
ISSN: 0392-856X
• SORBERA L A ET AL: "STRONTIUM RANELATE
TREATMENT AND PREVENTION OF
OSTEOPOROSIS BONE RESORPTION
INHIBITOR BONE FORMATION STIMULANT"
DRUGS OF THE FUTURE, BARCELONA, ES, vol.
28, no. 4, 1 April 2003 (2003-04-01), pages 328-335,
XP008036784 ISSN: 0377-8282
• SORBERA L A ET AL: "STRONTIUM RANELATE
TREATMENT AND PREVENTION OF
OSTEOPOROSIS BONE RESORPTION
INHIBITOR BONE FORMATION STIMULANT",
DRUGS OF THE FUTURE, PROUS SCIENCE, ES,
vol. 28, no. 4, 1 April 2003 (2003-04-01), pages
328-335, XP008036784, ISSN: 0377-8282, DOI:
10.1358/DOF.2003.028.04.726503

Remarks:
The file contains technical information submitted after
the application was filed and not included in this
specification

**Description**

**Field of the invention**

The present application relates to a composition which is a tablet, for medical use for a administration once daily comprising at least one specific strontium salt as claimed.

The invention also relates to the tablet for said use for treating a male suffering from diseases and conditions affecting metabolism and/or structural integrity of cartilage and/or bone. The invention also relates to the tablet for said use for preventing a cartilage and/or bone condition in a subject, and for use in the treatment and/or prophylaxis of secondary osteoporosis.

**Background of the invention**

Osteoporosis is the most common form of metabolic bone disease in humans. It is a condition, which affects a very large number of people all over the world, and as the number of elderly people is set to rise dramatically in the coming decades in most 15 countries, the prevalence and impact of osteoporosis will also increase. The disease is characterized pathologically by an absolute decrease in the amount of bone mass and the structural quality of bone, and clinically by increased susceptibility to fractures. In fact, osteoporosis is the most significant underlying cause of skeletal fractures in late middle aged and elderly women.

In general, there are two types of osteoporosis: primary and secondary. Secondary osteoporosis is the result of an identifiable disease process, treatments or therapeutic agents. However, approximately 90% of all osteoporosis cases are idiopathic primary osteoporosis. Such primary osteoporosis includes postmenopausal osteoporosis, age-25 associated osteoporosis (affecting a majority of individuals over the age of 70 to 80), and idiopathic osteoporosis affecting middle-aged and younger men and women.

The mechanism of bone loss in osteoporosis is believed to involve an imbalance in the process of bone remodeling. Bone remodeling occurs throughout life, renewing the 30 skeleton and maintaining the strength of bone. This remodeling is mediated by specialized cells of the bone tissue, called "osteoclasts" and "osteoblasts". Osteoclasts (bone dissolving or resorbing cells) are responsible for the resorption of a portion of bone within the bone matrix, during the resorption process. After resorption, the osteoclasts are followed by the appearance of osteoblasts (bone forming cells), which then refill the 35 resorbed portion with new bone.

The formation of the two cell types as well as their activity in bone is usually tightly coupled and well regulated in order to maintain the skeletal balance and structural integrity of the bones. However, in people with osteoporosis an imbalance in this remodeling process develops, resulting in loss of bone at a rate faster than the accretion of bone.

The single most important risk factor for osteoporosis is oestrogen deficiency occurring naturally at the menopause. The decline in endogenous oestrogen production leads to an elevated metabolic activity in the bone tissue where the increase in osteoclast mediated bone resorption surpass the more modest increase in bone formation resulting in a net loss of bone. The actual number of people affected will grow at a rate greater than simple population growth rates, because the aging of the population is disproportionately increasing the older segment of the population, while the age for the onset of menopause has remained constant. In the last decades there has also been a substantial advance in the ability to predict and monitor osteoporosis, as methods for measurement of bone mineral density (BMD) has improved and new specific biochemical markers of bone resorption and formation has been developed and made available for routine clinical use. New pharmaceutical agents for treatment and/or prevention of osteoporosis have also been developed. The majority of these treatments are either based on substituting the lost endogenous estrogen either in the form of hormone replacement therapy (HRT) or selective estrogen receptor modulators (SERM), or they belong to the class of compounds called bisphosphonates. SERM's and especially HRT can only be administered to female subjects as administration of estrogen and estrogen like substances to a male will be associated with unwanted hormonal effect. Furthermore even in women the use of SERMs and especially HRT is associated with significant side effects, such as increased risk of cancer and cardiovascular disease, whereas bisphosphonates in addition to a potent antiresorptive effect also decreases bone formation to a similar extent, implying that they loose their therapeutic effect after few years of treatment. Thus, there is a need for agents, which are effective in the treatment and/or prophylaxis of osteoporosis

SORBERA LA ET AL: "STRONTIUM RANELATE TREATMENT AND PREVENTION OF OSTEOPOROSIS BONE RESORPTION INHIBITOR BONE FORMATION STIMULANT". DRUGS OF THE FUTURE, PROUS SCIENCE, ES, vol. 28, no. 4, 1 April 2003 (2003-04-01), pages 328-335, discloses the use of a soluble strontium salt of an organic acid (strontium ranelate, a synthetic non-naturally occurring thiophene-substituted amino acid) for the treatment of osteoporosis. In the studies discussed in said document, PREVOS and STARTOS replace with "strontium ranelate was administered" once a day, see table I, 1000 mg perorally, once daily. Said document furthermore describes that strontium ranelate has good bioavailability. Said document is therefore the closest prior art.

**Description of the invention**

The scope of the invention is limited by the appended claims.

In a first aspect of the invention, it concerns a composition which is a tablet, for medical use for a administration once daily comprising at least one specific strontium salt as claimed. The composition is intended for administration once daily. In a specific aspect of the invention, the strontium salt is characterized by having a water solubility of at the most about 200 g/l at room temperature and in a specific aspect, the strontium salt has a relatively low water solubility under physiological conditions (i.e. a solubility below 1 g/l at 40°C).

In a second aspect of the invention it relates to a pharmaceutical composition containing a strontium salt, wherein the composition is adapted to release the Sr salt in such a manner that the amplitude (difference between peak and nadir) of the plasma concentration relative to the peak level should be less than about 40% such as, e.g. less than about 35%, less than about 30%, less than about 25%, less than about 20%, less than about 15% or less than about 10% after administration of the composition to a subject once daily for a time period of 7 days or more. In a preferred aspect, the time period is 7 days.

In one embodiment of the invention the plasma concentration may fluctuate from about 16.2 +/- 3 mg/l to 20.0 +/- 2.3 mg/l Sr after administration of a pharmaceutical composition comprising a daily dose of approximately 650 mg ionic strontium.

It is contemplated that therapy with Sr salts would be significantly improved by reducing the frequency of administration. Firstly, it is possible to reduce or minimize unwanted side-effects and moreover, it is possible to achieve a plasma level that is constant or substantially constant during a prolonged period of time, i.e. leading to a reduction in the amplitude between the peak and the nadir values of the plasma concentration. Accordingly, the patient will potentially have a more efficient treatment with a sustained treatment effect (e.g. continuous anti-osteoporotic effect) during the treatment period.

A suitable *in vitro* method for determining whether a specific composition has suitable properties with respect to controlled release of the Sr salt is an *in vitro* dissolution test as described in Ph. Eur. Thus, a controlled release composition according to the invention - when tested in an *in vitro* dissolution test - releases strontium ion from the Sr salt containing pharmaceutical composition in the following manner:

within the first 30 min of the test at the most about 10% w/w of the Sr ion is released
within the first 4 hours of the test at the most about 70% w/w of the Sr ion is released
within the first 14 hours of the test about 70% w/w or more of the Sr ion is released.

The tablet used in the invention may be a composition, wherein the Sr salt is contained in a matrix that governs the release.

The composition may also be coated with a controlled release coating governing the release of the Sr containing compound.

Some of the known strontium salts (e.g. strontium chloride, strontium hydroxide) have a very high water-solubility (i.e. above 200 g/l in water at room temperature 20 - 25°C). Irrespective of their water-solubility such strontium salts may be incorporated into a controlled release composition for once daily administration. However, in a specific embodiment of the invention the water-solubility of the strontium salt is at the most about 200 g/l such as, e.g. at the most about 150 g/l, at the most about 100 g/l, at the most about 75 g/l, at the most about 50 g/l, at the most about 25 g/l, at the most about 10 g/l, at the most about 5 g/l, at the most about 2.5 g/l, or at the most about 1 g/l at room temperature (20-25 °C).

Furthermore, the invention relates to such a composition, wherein the amount of the Sr salt is adjusted so that the composition is suitable for administration once or twice daily.

As mentioned above, such a composition may be suitable for administration once daily, e.g. at bedtime. It is known that bone resorption is higher during the night than during daytime, why the administration of an amount of Sr at bedtime could prove to be favorable compared to administration of a similar amount of Sr in the morning. As shown in the examples herein, a number of strontium salts, i.e. those that has a water solubility of less then 200 g/l (as mentioned above) have a delayed appearance of peak concentration of the ionic strontium compared e.g. to that of the highly water soluble strontium chloride. Accordingly, such salts are contemplated to be suitable for use in designing a controlled release pharmaceutical composition containing a strontium salt.

The daily dose of strontium ion may be at least about 0.01 g, such as, e.g. at least about 0.025 g, at least about 0.050 g, at least about 0.075 g, at least about 0.1 g, at least about 0.2 g, at least about 0.3 g, at least about 0.4 g or at least about 0.5 g or from about 0.01 to about 2 g such as, e.g., from about 0.1 to about 2 g, from about 0.1 to about 1 g, from about 0.15 to about 0.5 g, from about 0.3 to about 2 g or from about 0.3 to about 1 g.

In a specific embodiment the invention also relates to such a composition, comprising at least 0.5 g of Sr, defined as free ionic strontium, such as, e.g. at least 0.6 g, at least 0.7 g, at least 0.8 g, at least 0.9 g, at least 1.0 g, at least 1.1 g, at least 1.2 g, at least 1.3 g, at least 1.4 g, at least 1.5 g, at least 1.6 g, at least 1.7 g, at least 1.8 g, at least 1.9 g or at least 2.0 g daily.

Furthermore, the invention relates to such a composition for use in a method for the treatment and/or prophylaxis of a cartilage and/or bone disease and/or conditions resulting in a dysregulation of cartilage and/or bone metabolism in a mammal, such as e.g. a human female or male adult, adolescent or child, such as e.g. osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, for the improvement of implant stability and suitable for the maintenance or increase of energy level, for building up or strengthening muscle tissues and for weight gain, the method comprising administering a single daily dose of a Sr saltcomprising at least 0.7 g Sr, such as, e.g., at least 0.8 g, at least 0.9 g, at least 1.0 g, at least 1.1 g, at least 1.2 g, at least 1.3 g, at least 1.4. g, at least 1.5 g, at least 1.6 g, at least 1.7 g, at least 1.8 g, at least 1.9 g or at least 2.0 g.

*Strontium*

Previous studies have shown that various strontium compounds modulate bone loss in osteoporosis when present at levels higher than those required for normal cell physiology. The effect is believed to be due to a stimulatory effect of strontium on pre-osteoblastic cell replication, and a direct or matrix-mediated inhibition of osteoclast activity by strontium (Reginster, JY, Curr pharm Des 2002:8 (21):1907-16). In other words, strontium both works as an anti-resorptive and an anabolic agent. Various salts of strontium are known from the prior art, such as, e.g., strontium ranelate (distrontium salt of 2-[N,N-di(carboxymethyl)amino]-3-cyano-4-carboxymethylthiophene-5-carboxylic acid) described in EP-B 0 415 850. The ranelate part of the strontium compound, derived from ranelic acid, is unlikely to have any therapeutic effect towards cartilage or bone conditions per se.

The strontium salts as listed in the claim 1 may be in a composition as described above. The salts may be in hydrate, anhydrous, solvate, polymorphous, amorphous, crystalline, microcrystalline or polymeric form. In one embodiment of the invention only non-radioactive isotopes of Sr are used.

The organic acid may be selected from the group consisting of maleic acid, malonic acid, L- and D-glutamic acid, pyruvic acid, L- and D-aspartic acid, , alpha-ketoglutaric acid,

As mentioned above, the strontium salt for use according to the invention may be water soluble, having a water solubility of at least 0.1 g/l such as, e.g., in a range of from about 0.1 g/l to about 10 g/l, from about 0.2 g/l to about 5 g/l at room temperature exemplified by the salts listed above i.e. in a specific aspect of the invention the strontium salt has a water-solubility of at least 1 g/l, such as, e.g., at least 5 g/l, at least 10 g/l, at least 20 g/l, at least 30 g/l, at least 40 g/l, at least 50 g/l, at least 60 g/l, at least 70 g/l, at least 80 g/l, at least 90 g/l or at least 100 g/l measured at room temperature of (20-25 °C).

The daily dose of strontium may be at least about 0.01 g, such as, e.g. at least about 0.025 g, at least about 0.050 g, at least about 0.075 g, at least about 0.1 g, at least about 0.2 g, at least about 0.3 g, at least about 0.4 g or at least about 0.5 g or from about 0.01 to about 2 g such as, e.g., from about 0.1 to about 2 g, from about 0.1 to about 1 g, from about 0.15 to about 0.5 g, from about 0.3 to about 2 g or from about 0.3 to about 1 g.

*Synthesis of strontium salts*

This section on synthesis does not form part of the invention.

Organic strontium salts of carboxylic acid anions can be synthesized by a number of different pathways. A conventional method for preparation of such organic strontium salts is to utilise the reaction between and organic acid and strontium hydroxide in an aqueous solution. This neutralisation reaction of, e.g. fumaric acid and strontium hydroxide salt follows the following scheme:

$$Sr^{2+} (aq) + 2OH^- (aq) + HOOCCHCHCOOH(aq) \rightarrow Sr(OOCCHCHCOOXaq) + 2H_2O(t)$$

The suspension of dissolved strontium fumarate can then be induced to precipitate by sublimation of water and subsequent up-concentration of the salt. Crystals will slowly form and precipitate from the solution.

An alternative approach is to utilise the sodium or potassium salt of the appropriate carboxylic acid anion and strontium chloride. As all organic strontium salts will be less soluble than the highly soluble chloride salt, the organic strontium salt will precipitate under these conditions leaving NaCl and excess $SrCl_2$ in the solution. The equation below exemplifies this reaction scheme using as an example the reaction between $SrCl_2$ and sodium-fumarate.

$$Sr^{2+} (aq) + 2Cl^- (aq) + 2Na^+ (aq) + C_4H_2O_4^{2-} (aq) \rightarrow$$

$$Sr(OOCCHCHCOO(aq) + Cr (aq) + Na^+ (aq)$$

The present inventors have found that different strontium salts requires different synthesis pathways, and for some strontium salts we have identified optimized synthesis and manufacturing procedures. It has been found that synthesis of strontium salts of the di-carboxylic amino acids aspartate and glutamate (in either D- or L- form) is very difficult when following these conventional reaction pathways, and generally results in low yields and purity of the obtained crystalline salt. In order to facilitate large-scale manufacture of pure strontium salts of dicarboxylic amino acids to carry out the pharmaceutical use according to the present invention, the present inventors have studied various synthesis pathways of these particular strontium salts. Thus, it has surprisingly been found that synthesis of well defined and pure strontium glutamate in hexahydrate form is most convenient carried out with the free acid form of glutamate and strontium hydroxide and requires elevated temperatures, such as temperatures above 80°C, or more preferred 100°C or even 120°C or most preferred more than 130°C (see example 7, where manufacturing procedures for synthesis of organic strontium salts at high temperature are described).

Furthermore, we have found that addition of small volumes of alcohol can accelerate the crystal-formation of dissolved aqueous organic strontium salts. Examples of these synthesis procedures for organic strontium salts of relevance for the treatment and/or prophylaxis of bone disease are provided in the examples herein.

*Pharmaceutical compositions*

The invention also relates to pharmaceutical compositions as described above comprising at least one strontium compound. The pharmaceutical compositions according to the invention normally further comprise one or more physiologically acceptable excipients, i.e. a therapeutically inert substance or carrier.

The carrier may take a wide variety of forms depending on the desired dosage form and administration route.

The pharmaceutical composition is designed to release the active substance in the gastrointestinal tract, i.e. in a preferred aspect it is not intended for application to or absorption via the oral mucosa.

The composition is a tablet such as, e.g. conventional tablets, effervescent tablets, coated tablets, melt tablets or sublingual tablets, pellets, powders, granules, granulates, particulate material, solid dispersions or solid solutions.

. The tablet may be coated with a coating that enables release of at least part of the salt in proximal part of the small intestine, such as e.g. the duodenum and/or the proximal jejunum such as at least 50% w/w, at least 60% w/w, at least 65% w/w, at least 70% w/w, at least 80% w/w or at least 90% w/w of the total amount of the salt contained in the tablet.

The tablet may have a shape that makes it easy and convenient for a patient to swallow. The tablet may thus e.g. have a rounded or a rod-like shape without any sharp edges. Furthermore, the tablet may be designed to be divided in two or more parts.

The pharmaceutical compositions may be prepared by any of the methods well known to a person skilled in pharmaceutical formulation.

The pharmaceutically acceptable excipients may be e.g. fillers, binders, disintegrants, diluents, glidants, solvents, emulsifying agents, suspending agents, stabilizers, enhancers, flavors, colors, pH adjusting agents, retarding agents, wetting agents, surface active agents, preservatives, antioxidants etc. Details can be found in pharmaceutical handbooks such as, e.g., Remington's Pharmaceutical Science or Pharmaceutical Excipient Handbook. In those cases, where the pharmaceutical composition is intended for controlled release of the Sr containing compound, it may also comprise release controlling agents such as, e.g., material normally used in the formulation of matrix tablets (e.g. cellulose derivatives like hydroxypropyl methylcellulose and the like). Alternatively, the composition may be coated with a controlled release coating such as an enteric coating or a film coating. A suitable coating may be a substantially water-insoluble but water-permeable coating.

The invention relates to a controlled release pharmaceutical composition for oral use which is a tablet.

The term "tablet" is intended to embrace compressed tablets, coated tablets, matrix tablets, osmotic tablets, and other forms known in the art.

The term "multiparticulate" is intended to embrace a dosage form comprising a multiplicity of particles and/or granulates whose totality represents the intended therapeutically useful dose. The particles generally are of a diameter from about 50 microns to about 0.3 cm, with a preferred range of 100 $\mu$m to 1mm. Multiparticulates represent a suitable embodiment for use in scaling dosage forms release because they are amenable according to the weight of an individual subject (e.g. a mammal such as a human).

Also disclosed is a process for preparing controlled or delayed release dosage forms of a strontium salt e.g. comprising the steps of mixing or granulating the strontium salt together with one or more pharmaceutically acceptable excipients (selected from the group consisting of fillers, binders, disintegrants, release rate modifier etc.) to obtain a powder blend that can be further processes into e.g. matrix pellets or tablets or into pellets or tablets that are provided with a controlled release polymer coating controlling the release of the strontium salt.

In the case of delayed release embodiments, the dosage form can take the same forms as above, provided that the dosage form delivers the majority of its strontium salt to regions of the gastrointestinal tract distal to the duodenum. A variety of dosage form embodiments and/or structures may be used to achieve this goal, i.e. multiparticulates, beads, pellets or other particle dosage forms that may be compressed into a tablet.

The controlled or delayed release dosage forms of this invention can be widely implemented. Different embodiments include e.g. matrix systems, in which the strontium salt is embedded or dispersed in a matrix of another material that serves to retard the release of the active substance into an aqueous environment (i.e. the lumen fluid of the GI tract). When the strontium salt is dispersed in a matrix of this sort, release of the drug takes place principally from the surface of the matrix.

Thus the active substance is released from the matrix surface after it has diffused through the matrix or when the surface of the composition erodes and thus exposes the active substance. In some embodiments, both mechanisms can operate simultaneously. The matrix systems may be large, i.e., tablet sized (about 1 cm), or small (< 0.3cm). The system may be a single unit or multiple units, which are administered substantially simultaneously, or may comprise a plurality of particles, referred to herein as a multiparticulate. A multiparticulate can have numerous formulation applications. For example, a multiparticulate may be used as a powder for filling a capsule shell, or used per se for mixing with food to increase palatability.

Slowly-hydrating materials may also be used to give the desired release rate. The multiplicity of variables affecting release of the active substance from matrices permits flexibility in the design of compositions of different materials, sizes, and release times. Examples of modifications of strontium ion release profiles are within the scope of this invention.

A specific embodiment of the invention relates to a matrix multiparticulate comprises a plurality of strontium salt -containing particles, each particle comprising a mixture of the strontium salt with one or more appropriate pharmaceutically acceptable excipient selected to form a matrix capable of limiting the dissolution rate of the strontium salt into an aqueous medium. The matrix materials useful for this embodiment are generally water-insoluble materials such as waxes, cellulose, or other water-insoluble polymers. If needed, the matrix materials may optionally be formulated with water-soluble materials, which can be used as binders or as permeability modifying agents. Matrix materials useful for the manufacture of these dosage forms include microcrystalline cellulose such as Avicel (registered trademark of FIVIC Corp., Philadelphia, PA), including grades of microcrystalline cellulose to which binders such as hydroxypropyl methyl cellulose have been added, waxes such as paraffin, modified vegetable oils, carnauba wax, hydrogenated castor oil, beeswax, and the like, as well as synthetic polymers such as poly(vinyl chloride), poly(vinyl acetate), copolymers of vinyl acetate and ethylene, polystyrene, and the like. Water soluble binders or release modifying agents which can optionally be formulated into the matrix include water-soluble polymers such as hydroxypropyl cellulose (HPC), hydroxypropyl methyl cellulose (HPIVIC), Methyl cellulose, poly (N-vinyl pyrrolidinone) (PVP), poly(ethylene oxide) (PEO), poly(vinyl alcohol) (PVA), xanthan gum carrageenan,and other such natural and synthetic materials. In addition, materials that function as modifying agents include water-soluble materials such as sugars or salts. Preferred water-soluble materials include lactose, sucrose, glucose, and mannitol, as well as HPC, HPMC, and PVP.

A suitable process for manufacturing matrix multiparticulates is the extrusion/spheronization process. For this process, the active substance is wet massed with a binder, extruded through a perforated plate or die, and placed on a rotating disk.

The extrudate ideally breaks into pieces, which are rounded into spheres, spheroids, or rounded rods on the rotating plate.

A further preferred process for manufacturing matrix multiparticulates is the preparation of wax granules. In this process, a desired amount of the active substance is stirred with a wax to form a homogeneous mixture, which is cooled and then forced through a screen to form granules. Suitable matrix materials are waxy substances such as, e.g., hydrogenated castor oil and carnauba wax and stearyl alcohol.

A further process for manufacturing matrix multiparticulates involves using an organic solvent to aid mixing of the active substance with the matrix material. This technique can be used when it is desired to utilize a matrix material with an unsuitably high melting point that, if the material were employed in a molten state, would cause decomposition of the drug or of the polymeric matrix material. Alternatively, the active substance and matrix material may be combined with a solvent to dissolve the matrix material and the resulting solution (which may contain solid drug particles) is e.g. spray dried to form the particulate dosage form. This technique is preferred when the matrix material is a high molecular weight synthetic polymer such as any cellulose ether or cellulose ester. Solvents typically employed for the ethanol, isopropanol, ethyl acetate, and mixtures process include acetone, of two or more.

Once formed, the matrix multiparticulates may be blended with compressible excipients such as.lactose, microcrystalline cellulose, calcium phosphate, and the like and the blend is compressed to form a tablet. Disintegrants such as sodium starch glycolate or crosslinked poly(vinyl pyrrolidone) are also usefully employed. Tablets prepared by this method disintegrate when placed in an aqueous medium (such as the GI tract), thereby exposing the multiparticulate matrix, which releases the strontium salt and/or the ionic form of free strontium from the composition.

A further embodiment of a matrix system has the form of a hydrophilic matrix tablet containing the active substance and an amount of hydrophilic polymer sufficient to provide a useful degree of control over the dissolution of the strontium salt. Hydrophilic polymers useful for forming the matrix include hydroxypropylmethyl cellulose (HPMC), hydroxypropyl cellulose (HPC), poly (ethylene oxide), poly(vinyl alcohol), xanthan gum, carbomer, carrageenein, and zooglan. A suitable material is HPMC. Other similar hydrophilic polymers may also be employed. Using a lower molecular weight polymer may increase the dissolution rate. The dissolution rate may also be controlled by the use of water-soluble additives such as sugars, salts, or soluble polymers. Examples of these additives are sugars such as lactose, dextrose, cyclo-dextrose, sucrose, or mannitol, salts such as NaCl, KCl, NaHCO3, and water soluble polymers such as PNVP or PVP, low molecular weight, HPC or HIVIPC or methyl cellulose. In general, increasing the fraction of soluble material in the formulation may increase the release rate. A matrix tablet typically comprises about 20 to 90% by weight of the active substance and about 10 to 80% by weight of polymer.

A suitable matrix tablet comprises, by weight, about 50% to about 80% the active substance about 15% to about 35% HPMC, 0% to about 35% lactose, 0% to about 15% PVP 0% to about 20% microcrystalline cellulose, and about 0.25% to about 2% magnesium stearate.

The matrix systems as a class often exhibit non-constant release of the drug from the matrix. This result may be a consequence of the diffusive mechanism of drug release, and modifications to the geometry can be used to make the release rate of the drug more constant as detailed below.

In a further embodiment, a matrix tablet may be coated with an impermeable coating and an orifice (for example, a circular hole or a rectangular opening) is provided by which the content of the tablet is released.

In a suitable embodiment a tablet or capsule is coated with an impermeable material on part of its surface, e.g. on one or both tablet faces, or on the tablet radial surface.

The dosage form may be coated with a coating that modulates the release of the active substance. By "impermeable material" is meant a material having sufficient thickness and impermeability to the active substance such that no significant transport thereof can take place through the material during the time scale of the intended drug release (i.e., several hours to about a day). Such a coating can be obtained by selecting a coating material with a sufficiently low diffusion coefficient for the active substance and applying it sufficiently thickly.

Materials for forming the impermeable coating of these embodiments include substantially all materials in which the diffusion coefficient of the active substance is suitable. Preferred coating materials include film-forming polymers and waxes. Especially preferred are thermoplastic polymers, such as poly(ethylene-co-vinyl acetate), poly(vinyl chloride), ethylcellulose, and cellulose acetate. These materials exhibit the desired low permeation rate of the active substance when applied as coatings.

A further controlled release matrix system comprises the active substance dispersed in a hydrogel matrix. This embodiment differs from the hydrophilic matrix tablet discussed above in that the hydrogel of this embodiment is not a compressed tablet of erodible granular material, but rather a monolithic polymer network. As known in the art, a hydrogel is a water-swellable network polymer. Hydrogels are preferred materials for matrix devices because they can absorb or be made to contain a large volume fraction of water, thereby permitting diffusion of solvated drug within the matrix.

Diffusion coefficients of drugs in hydrogels are characteristically high, and for highly water-swollen gels, the diffusion coefficient of the drug in the gel may approach the value in pure water. This high diffusion coefficient permits practical release rates from relatively large devices (i.e., it is not necessary to form microparticles). Preferred materials include hydrophilic vinyl and acrylic polymers, polysaccharides such as calcium alginate, and poly(ethylene oxide). Especially suitable are poly(2-hydroxyethyl. methacrylate), poly(acrylic acid), poly(methacrylic.acid), poly(N-vinyl pyrolidinone), poly(vinyl alcohol) and their copolymers with each other and with hydrophobic monomers such as methyl ethacrylat el vinyl acetate, and the like. Also preferred are hydrophilic polyurethanes containing large poly(ethylene oxide) blocks. Other preferred materials include hydrogels comprising interpenetrating networks of polymers, which may be formed be addition or by condensation polymerization, the individual monomers components may comprise hydrophilic and hydrophobic groups.

Other coating materials include ethyl cellulose, cellulose acetate and cellulose acetate butyrate. The polymer may be applied as a solution in an organic solvent or as an aqueous dispersion or latex. The coating operation may be conducted in standard equipment such as a fluid bed coater, a Wurster coater, or a rotary bed coater.

If desired, the permeability of the coating may be adjusted by blending of two or more materials. A particularly useful process 1 or tailoring the porosity of the coating comprises adding a pre-determined amount of a finely-divided water-soluble material, such as sugars or salts or water soluble polymers to a solution or dispersion (e.g., an aqueous latex) of the membrane-forming -polymer to be used. When the dosage form is ingested into the aqueous medium of the GI tract, these water soluble membrane additives are leached out of the membrane, leaving pores which facilitate release of the drug. The membrane coating can also be modified by the addition of plasticizers, as known in the art.

Above are mentioned specific examples of the amounts of compounds administered. However, it will be understood that the amount of the compounds actually administered will be determined by a physician in light of the relevant circumstances including the condition to be treated, the choice of compounds to be administered, the age, weight, and response of the individual patient, the severity of the patient's symptoms and/or signs, and the chosen route of administration. While the present compounds are preferably administered orally, the compounds may also be administered by any other suitable route.

*Treatment of males*

Contrary to popular belief, osteoporosis is not just a disease of women. Males are not as resistant to osteoporosis as once thought, and the classical age-related increase in fractures seen in women is also evident in men. One of the main reasons why osteoporosis is not as common in males as in women is the larger skeleton of the males. Other factors include the shorter life expectancy, later onset and slower progress of bone loss in men, and the absence of rapid bone loss that affects women as a result of cessation of endogenous oestrogen production at the menopause.

However, as understanding of the pathophysiology of the disease has increased in recent years, it's been recognized that male osteoporosis represents an important public health issue. In the United States alone, up to 5 million men suffer from osteoporosis, and their number is rising. Almost 30-40% of patients develop so-called "idiopathic" osteoporosis at a young age, in the absence of any detectable cause, whereas others have multiple evident secondary reasons for bone loss, including glucocorticoid excess, hypogonadism, alcohol abuse, smoking, renal tubular disease with calcium wasting, or other liver or bowel diseases.

Accordingly, the invention relates to the tablet for use as claimed for the treatment and/or prophylaxis of a cartilage and/or bone disease and or conditions resulting in a dysregulation of cartilage and/or bone metabolism in a male subject, such as e.g. osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, for the improvement of implant stability and for the maintenance or increase of energy level, for building up or strengthening muscle tissues and for weight gain, the method comprising administering a Sr salt in an amount and frequency that may give a daily dose of from about 0.25 to about 1.5 g ionic free $Sr^{2+}$ such as, e.g. from about 0.30 g to about 1.5 g, from about 0.40 g to about 1.40 g, from about 0.50 g to about 1.30 g, from about 0.60 g to about 1.20 g, from about 0.60 g to about 1.0 g or from about 0.60 g to about 0.8 g.

The Sr salt is administered orally, and is contained in a pharmaceutical composition as defined above.

*Prophylaxis*

Some of the drugs used today for the treatment of diseases and conditions affecting metabolism and/or structural integrity of bone and/or cartilage may have a therapeutic effect on the condition, but at the same time many of the drugs used are associated with severe side effects. An example of a group of drug substances with severe side effects are the bisphosphonates, which appear to have detrimental side effects, such as, e.g., the potential of inhibiting bone formation as well as resorption and poor absorption via oral administration. Furthermore, they are known to cause G.I. irritation and to have extremely long half-lives in bone. Therefore, the subject in need of treatment potentially should have a minimal exposure to such compounds. Accordingly, such drug substances are not suitable for prophylactic treatment.

As there are no known side effects associated with the administration of strontium in the doses suitable for prophylaxis, strontium will probably be very useful for the prevention of cartilage and/or bone conditions. Accordingly, the invention relates to the tablet for use as claimed is a method for the treatment and/or prophylaxis of a cartilage and/or bone disease and/or conditions resulting in a dysregulation of cartilage and/or bone metabolism in a mammal, such as e.g., a human female or male adult, adolescent or child, such as, e.g., osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, suitable for the improvement of implant stability and for the maintenance or increase of energy level, for building up or strengthening muscle

tissues and for weight gain, the method comprising administering a Sr containing compound.

The measurement of BMD, bone mineral density, or other forms of radiographic assessment of bones or joints can be used to establish or confirm a diagnosis of diseases and conditions affecting metabolism and/or structural integrity of cartilage and/or bone, such as, e.g. osteoporosis and osteopenia. The BMD value may also be used for determining whether a prophylactic treatment should be initiated.

Several techniques are available to measure BMD non-invasively. Bone densitometry is the best available method for diagnosing osteoporosis and osteopenia and other bone conditions, and for identifying subjects at risk for developing a bone condition. In bone densitometry the amount of bone mineral present is measured, which is an important determinant of bone strength. The traditional bone densitometry methods are based on X-ray absorptiometry, such as, e.g. dual-energy x-ray absorptiometry, single-energy X-ray absorptiometry and radiographic absorptiometry. In addition to these approaches, qualitative computed tomography, which utilizes a computed tomography scan, can be used to calculate BMD. Another bone densitometry method is quantitative ultrasound, which is fairly inexpensive, portable and radiation free. Here the bone part to be measured is positioned between two ultrasound transducers, and bone mass is determined by the transmission of sound waves passing through the bone; the fewer that pass, the denser the bone.

In order to standardize values from different densitometers, and to give a value that easily can be used for diagnosis, the BMD value may be used to calculate values called the T-score and the Z-score.

The T-score is considered the most clinically relevant value, and describes the subject's BMD relative to the mean BMD for young adult normal women or men, respectively, expressing the difference as number of standard deviations (SDs). In the case of females, if the T-score of a subject is less than 1 SD below the mean of young adult normal women, the BMD is considered normal. For each SD below the mean bone mass of young adult normal women, the risk of fracture increases by approximately 1.5 to 3 fold, and below 2 SDs, it increases exponentially.

The Z-score compares the subject's BMD to the mean BMD for males or females having the same age as the subject. Among older adults, however, a low BMD is common, so comparison with age-matched norms may be misleading, i.e. an 80-year-old subject may have a Z-score that compares favorably with age-matched controls, but nevertheless, like the average patient in this age group, the subject may be at risk of experiencing fracture(s).

In a method used in the invention for preventing a cartilage and/or a bone condition, subjects who are at risk of developing such a condition may be identified by calculating the subjects' T-scores. Accordingly, the present invention relates to a method wherein the subject is a female having a bone mineral density, BMD, of more than 1 SD below the adult female mean.

In another method the Z-score is calculated, i.e. the present invention relates to the tablet for use as claimed in a prophylactic method, wherein the subject is a female having a BMD below the female mean for women of the same age. If the female belongs to an age-group wherein the average females may have a higher risk of bone fracture, a prophylactic treatment may be considered, even though the female has a BMD at or above the value of the female mean for women of the same age.

The invention also relates to the tablet for use as claimed in a prophylactic method as described above, wherein the subject is a male having a BMD of more than 1 SD below the adult male mean.

Furthermore, the invention also relates to the tablet for use as claimed in a method wherein the subject is a male having a BMD below the adult male mean for men of the same age. If the male belongs to an age-group wherein the average males may have a higher risk of bone fracture, a prophylactic treatment may be considered, even though the male has a BMD at or above the value of the male mean for men of the same age.

The invention also relates to a the tablet for use as claimed in prophylactic method, wherein the subject is a 20 year or older such as, e.g., 25 years or older, 30 years or older, 35 years or older, 40 years or older, 45 years or older, or 50 years or older female.

Another way of assessing the status of bone and cartilage is provided by dynamic biochemical markers, which reflect the turnover of either cartilage or bone. In comparison to BMD or other similar static measurements, which provide a measure of the current status of bone and/or cartilage, a specific biomarker can provide a measure of the current turnover of the tissue from which the marker is derived. This provides a dynamic monitoring of therapeutic effects and it also enables a prediction of progression of a disease or condition affecting bone and/or cartilage turnover. As an example it has been demonstrated in several studies that a specific marker of bone resorption, C-telopeptide derived collagen type I fragments, CTX, provides a BMD independent predictor of subsequent fracture risk, with similar potency as BMD measurements alone. Furthermore, the measure of fracture risk provided by CTX measurements is additive to the measure provided by BMD determinations, and thus individuals with both a low BMD, and a high bone turnover as indicated by elevated CTX levels are more at risk for sustaining skeletal fracture than individuals with only elevated BMD or decreased CTX. Similar data has been obtained with a specific marker of cartilage derived collagen type II fragments, CTX-II. Accordingly subjects at risk of developing pathological deterioration of bone and/or cartilage may be defined by measurement of specific biomarkers of either bone or cartilage metabolism. Similar to BMD measurements, the biomarker measurements may be expressed in T-scores or Z-scores related to relevant reference populations, or the values may simply be expressed relative to pre-defined cut-of levels.

The invention also relates to a the tablet for use as claimed in prophylactic method as described above, wherein the subject is a male having a biomarker level of a bone resorption marker such as CTX or NTX of more than 1 SD above the adult male mean.

Furthermore, the invention also relates to a the tablet for use as claimed in method wherein the subject is a male having a bone resorption marker such as CTX or NTX above the adult male mean for men of the same age. If the male belongs to an age-group wherein the average males may have a higher risk of bone fracture, a prophylactic treatment may be considered, even though the male has a bone resorption marker such as CTX or NTX below the mean levels of the given marker for men of the same age.

The invention also relates to a the tablet for use as claimed in prophylactic method, wherein the subject is a 20 year or older such as, e.g., 25 years or older, 30 years or older, 35 years or older, 40 years or older, 45 years or older, or 50 years or older female.

Furthermore, the invention relates to a combined use of BMD measurement and one or more biomarkers for definition and/or prediction of individuals of risk for progression of a disease or condition affecting bone and/or cartilage turnover.

Estrogen plays an important role in bone health, as estrogen protects the bones by preventing the skeletal system from elevation in bone turnover, which results in imbalance between formation and resorption of bone and subsequent skeletal deterioration. When the level of estrogen decreases after menopause, more bone is resorbed than is built. Women who do not take any form of medication preventing bone loss may lose as much as 3% to 5% of their bone mass in each of the 5 years following menopause, and by e.g. age 70, the bones can weigh 30% to 50% less than before menopause.

Accordingly, even though a female at the onset of menopause may have a BMD level and/or level of specific biomarkers of bone and/or cartilage turnover within the normal range (i.e. as defined by the T score), it may be beneficial to initiate a treatment for preventing the development of a bone condition in the future. Thus, the invention relates to a the tablet for use as claimed in method as described above, wherein the subject is a peri-menopausal female or a female with recent onset of menopause.

Furthermore, the invention relates to a the tablet for use as claimed in method, wherein the subject is a female who is about 6 months or more beyond the onset of menopause.

The invention also relates to a the tablet for use as claimed in method for prophylaxis of a cartilage and/or bone condition in males, wherein the subject is a 20 year or older such as, e.g., 25 years or older, 30 years or older, 35 years or older, 40 years or older, 45 years or older, 50 years or older, 55 years or older, 60 years or older, 65 years or older, or 70 years or older male.

*Secondary osteoporosis*

Even though 90 % of all osteoporosis cases are idiopathic primary osteoporosis, there also exists a need for preventing and/or treating secondary osteoporosis, which is the result of an identifiable disease process or agent. Accordingly, the invention relates to a the tablet for use as claimed in method for treating and/or preventing secondary osteoporosis in a subject, the method comprising administering an effective amount of a Sr salt to the subject.

Secondary osteoporosis may be induced by endocrine diseases and/or metabolic causes, such as, e.g. hypogonadism, hypercortisolism, hyperprolactinemia, anorexia nervosa, mastocytosis, porphyria, diabetes mellitus type I, primary or secondary hyperparathyroidism, hyperthyroidism, acromegaly, Cushing's syndrome, acidosis, Guacher's disease, hemochromatosis, androgen insensitivity and pregnancy.

The secondary osteoporosis may also be induced by nutritional conditions, such as, e.g. malabsorption, malnutrition, chronic hepatic disease, vitamin D deficiency, calcium deficiency, resections of parts of the gastrointestional tract (e.g. gastrectomy), smoking and alcohol abuse.

The administration of certain drug substances may also lead to secondary osteoporosis. Examples of such drugs substances are e.g. corticosteroids (including inhaled corticosteroids), heparin, anti-epileptic drugs (e.g. phenytoin), gonadotrophin releasing hormone analogs, loop diuretics, phenobarbital, anti-neoplastic agents/immunosuppressants (e.g. methotrexate and cyclosporin), thyroid hormones, depo-medroxyprogesterone acetate, calcineurin-calmodulin phosphatase inhibitors such as, e.g. Tacrolimus, and aromatase inhibitors, such as Formestane, Exemestane, Aminoglutethimide, Fadrozole, Rogletimide, Anastrozole, Letrozole and Vorozole.

A disturbed collagen metabolism and/or diseases of the connective tissue may also be the cause of secondary osteoporosis. Examples of such disorders are e.g. osteogenesis imperfecta, homocysteinuria, rickets, Ehlers-Danlos syndrome and Marfan's syndrome.

Bone marrow diseases such as, e.g myeloma, thalassemia and leukemia may also be the cause of secondary osteoporosis. Also rheumatologic/inflammatory diseases such as, e.g. systemic lupus erythematosus, ankylosing spondylitis and rheumatoid arthritis may cause secondary osteoporosis.

In children and adolescents, causes of secondary osteoporosis include juvenile arthritis, juvenile rheumatoid arthritis, juvenile chronic arthritis, childhood malignancy, neuromuscular diseases such as cerebral palsy, spina bifida and muscular dystrophy, familial dysautonomia, fibrous dysplasia, juvenile Paget's disease (osteoprotegerin deficiency), familial idiopathic bone pain and isolated hyperphosphatasemia. Other potential causes of secondary osteoporosis in children and adolescents are excessive exercise, amenorrhea, dermatomyositis, asthma, inflammatory bowel disease, such as Crohn's disease, and muscular dystrophy.

Other general causes of secondary osteoporosis may be hypophosphatasis, immobilization, cystic fibrosis, renal insufficiency, hypercalciuria, chronic obstructive pulmonary disease, mastocytosis, depression, spinal cord injury, sarcoidosis, malignancy, lymphoplasmacytoid lymphoma, organ transplantation and surgical as well as chemical castration of males (including, but not limited to the use of anti-androgens and/or gonadotropin releasing hormone analogues).

*Prophylaxis of secondary osteoporosis*

As mentioned above, some drugs may be the cause of secondary osteoporosis. In order to prevent the development of drug induced secondary osteoporosis in a subject, it may be beneficial to administer a prophylactic amount of Sr as part of the same treatment regimen as the administration of the drug substance.

Thus, the invention relates to a the tablet for use as claimed in method for preventing drug induced secondary osteoporosis in a subject, the method comprising administering to the subject a prophylactic amount of a Sr salt before, during or after treatment of the subject with the drug substance that induces osteoporosis.

The administration may take place substantially simultaneously with administration of the drug substance that induces osteoporosis, and the Sr salt and the drug substance that induces osteoporosis may be contained in the same pharmaceutical composition.

Accordingly, the invention relates to the tablet for the use as claimed comprising a Sr salt and a drug substance that induces osteoporosis together with a pharmaceutically acceptable excipient.

The Sr salt and the drug substance may also be administered simultaneously in separate, co-administered compositions. When two separate formulations are being co-administered, each formulation, especially those for use by the oral route, may be color-coded or otherwise easily identifiably labeled in order to avoid confusion by the subject or physician.

*Other aspects of the invention*

In some embodiments of the invention, the inventors have found that it is preferable if ranelate, if present at all, may be present in an amount of less than 5% w/w of the total amount of strontium.

Also disclosed is a kit used in improved fracture healing after traumatic or atraumatic fracture, where the fracture e.g. may be one of the following traumatic or atraumatic fractures: fracture to the distal radius, such as e.g. a Colle's fracture or a Smiths fracture, a fracture of the femur, such as e.g. the proximal femur, such as e.g. a cervical fracture, a trochanteric fracture or a subtrochanteric fracture.

The improved fracture healing may be defined in terms of reduction of the time a patient will require a plaster, reduction of the time to healing as defined on a X-ray, reduction in the time to fracture stability, improvement of callus formation as viewed by X-ray, reduction in time before appearance of callus formation as viewed by X-ray and/or reduction in time for regaining full or near-full mobility or physical activity level.

Other embodiments of the invention appear from the appended claims. The details and particulars described above and below and relating to the compounds and compositions according to the invention apply *mutatis mutandis* to the other aspects of the invention.

**Legends to figures**

Figure 1. X-ray diffractogram of crystals of strontium glutamate hexahydrate prepared by the method as described in example 7.

Figure 2. X-ray diffractogram of crystals of strontium malonate prepared by the method as described in example 7. The malonate salt of strontium have not previously been characterized and comprise a new crystallographic structure, but it is apparent from the stable baseline, and well defined spacing of diffraction peaks, that the crystal form of the malonate salt is homogeneous and pure.

Figure 3. Results of the optimisation experiments for strontium glutamate synthesis outlined in table 6. The influence on the yield of the synthesis of strontium glutamate was investigated by varying four parameters. (Yields above 100% indicate incomplete drying).

Figure 4: Plot of serum strontium concentrations measured in rats given a single dose of strontium as indicated in the upper part of each panel. The data points represent mean and standard deviation for each measuring point. Pre-dosis represent corresponding samples taken from animals treated with vehicle alone

The invention is further illustrated in the examples that are not intended to limit the invention in any way.

## Examples

**Example 1** (Reference)

**General method for preparation of crystalline salts of strontium by precipitation from dissolved strontium chloride and dissolved sodium salts of the appropriate carboxylic anions**

In a glass-beaker of 100 mL volume, 5 g of the sodium salt of the carboxylic acid was dissolved in a small volume of water that was slightly heated at temperatures not greater than 30-50 °C. The final volume was 25-50 mL. In another beaker 10 g of $SrCl_2$ ($SrCl_2$ hexahydrate, Sigma-Aldrich 43,966-5) was dissolved in 100 mL of water. This latter solution was slowly decanted into the first solution of the dissolved sodium salt. The transfer continued until an initial cloudiness was observed, which resulted in a total volume of 50-100 mL. The solution was allowed to rest at room temperature (22-24 °C) for several days until significant amounts of crystallized precipitate of the organic strontium salt appeared.

The reaction that proceeds is exemplified by the reaction between strontium ions and sodium fumarate (reaction schemes (a) and (b)):

$$NaOOCCHCHCOONa(s) + H_2O(l) \rightarrow {}^-OOCCHCHCOOH(aq) + 2Na^+(aq) + OH^-(aq) \quad \text{(a)}$$

$$^-OOCCHCHCOOH(aq) + Sr^{2+}(aq) \rightarrow Sr(OOCCHCHCOO)(aq) + H^+(aq) \quad \text{(b)}$$

In order to accelerate the crystallisation, we have found that addition of small volumes of ethanol, such as from 5 -10 vol/vol % to 50 - 60 % vol/vol induces a significant acceleration of the precipitation of the desired strontium salt. Addition of ethanol is of special importance in the synthesis of strontium salts with solubility exceeding 2 g/l at room temperature (22-24°), and will thus provide a substantial benefit for the synthesis of strontium salts of L-aspartate, L-glutamate and lactate. In order to reach the required product within a short period, it was essential to observe an initial crystallisation or an initial dimness in the solution right from the first stage.

After the precipitation, the solution was filtered on a Büchner funnel using a suction flask and the crystals were flushed in small volumes of ethanol. Crystals of some of the salts were very soluble, so in order to improve the yield of crystals, the solution was allowed to rest longer, such as at least 30 - 60 min. Repeated crystallisation resulted in yields of approx. 50%. Strontium salts of L-aspartate and of lactate were very soluble, with solubility exceeding 25 g/l in water at room temperature.

The lactate and L-glutamate salts of strontium were precipitated from solutions with an excess of strontium chloride and large crystals of the lactate salt were achieved by slow evaporation of the solvent.

**Example 2** (Reference)

**General method for preparation of crystalline salts by neutralisation of carboxylic acids with strontium hydroxide**

A small amount of the organic acid proper (0.75 - 3 g, see table below) was dissolved in water by heating to temperatures between 30°C - 50° C. Then, strontium hydroxide (Sigma Aldrich, $Sr(OH)_2*8H_2O$, MW 265.71, CAS no. 1311-10-0, approx. 10 g/L) was slowly added. Then, a magnetic stirring rod was added and the stirring and gentle heating (i.e. 30 - 50°C) of the suspension was started. After some time, the solution clarifies and all the solid material dissolves. The heating is maintained, and after three hours of incubation, the solution is filtered while hot on a Büchner funnel. Very small amounts of impurities were left in the filter.

The filtrate was subsequently allowed to cool at room temperature overnight, which resulted in growth of fine-powdered crystals of the desired strontium salt. Further purifications of the salts can be performed by repeated re-crystallizations (table 2).

Table 2: Amounts of start reagent used for organic strontium salt synthesis and recoveries in the synthesis of eight specific organic strontium salts following the general reaction pathway with free-acid forms of the anion, and strontium hydroxide.

| Strontium salt of (free acid used): | Sr(OH)$_2$ *8H$_2$O | Free acid | Amount obtained | Recovery* | Melting Temp. | Solubility | Crystal structure |
|---|---|---|---|---|---|---|---|
| Fumarate[1] | 2.044 g | 1.140 g | 0.999 g | 99 % | >380°C | Yes | No |
| α-ketoglutarate [2] | 2.017 g | 1.441 g | 0.828 g | 72 % | >380°C | Yes | No |
| succinate | 2.098 g | 1.177 g | 0.958 g | 92 % | 230°C | Yes | Yes |
| L-Ascorbate[3] | 2.094 g | 1.805 g | 2.005 g | 15 % | >380°C | Yes | No |
| L-Glutamate | 2.017 g | 1.453 g | 0.175 g | 15 % | >380°C | Yes | Yes |
| Citrate | 2.057 g | 1.918 g | 1.123 g | 48 % | >380°C | Yes | Yes |
| D-Aspartate | 2.190 g | 1.316 g | 0.167 g | 14 % | >380°C | No | No |
| Tartrate | 2.070 g | 1.502 g | 2.005 g | 129 % | >380°C | Yes | Yes |
| Notes *) Recovery calculated in % of the strontium content in Sr(OH)$_2$*8H$_2$O. | | | | | | | |

1) Fumaric acid is insoluble in water, and ethanol is added to the suspension until complete solubilization is achieved. The synthesis is continued with this material. 2) The strontium-AKG salts has a slight brownish appearance 3) In addition to the indicated amounts of strontium hydroxides and L-ascorbate an additional 4.087g SrCl$_2$*6H$_2$O solubilized in water is added to the reaction mixture.

**Example 3**

**Determinations of solubility of organic strontium salts**

*Synthesis of strontium salts*

The great majority of strontium salts could be obtained by reacting the sodium salt of the organic acid with strontium chloride following the general synthesis method described in example A. However, strontium citrate, strontium tartrate, strontium succinate and strontium α-ketoglutarate for the solubility investigations was obtained by synthesis from the free acid forms of the carboxylic acid and strontium hydroxide as described in example 2. Strontium glutamate was obtained as described in example 4, using an incubation temperature of 100°C for obtaining pure and homogeneous hexahydrate crystals of strontium glutamate. Detailed investigations of solubility were carried with the strontium salts listed in table 3 below:

Table 3: Overview of strontium salts used in investigation of solubility. MW indicates the molecular weight of the homogeneous crystalline form of the salt with the indicated amount of crystal water and % Sr gives the molar percentage that strontium constitutes of this crystalline form

| Strontium salt | MW | %Sr |
|---|---|---|
| Sr-ranelate (*7H$_2$O) † | 639.6 | 27.4 |
| SrCl$_2$ (*6H$_2$O) † | 266.6 | 32.9 |
| Sr-fumarate (*6H$_2$O) † | 309.7 | 28.3 |
| Sr-L-glutamate (*6H$_2$O) | 340.7 | 25.7 |
| Sr-a-ketoglutarate (*6H$_2$O) | 339.7 | 25.8 |
| Sr-aspartate (*3H$_2$O) | 272.7 | 32.1 |
| Sr-succinate (*6H$_2$O) | 311.7 | 28.1 |

(continued)

| Strontium salt | MW | %Sr |
|---|---|---|
| Sr-ascorbate (*6H$_2$O) † | 545.8 | 16.1 |
| Sr-maleate (*6H$_2$O) | 309.7 | 28.3 |
| Sr-malonate (*1H$_2$O) | 207.7 | 42.2 |
| Sr-pyruvate (*6H$_2$O) | 369.7 | 23.7 |
| Sr-tartrate (*6H$_2$O) † | 343.7 | 25.5 |
| Sr-citrate (*6H$_2$O) † | 749.1 | 35.1 |
| † Reference compounds | | |

The solubility of the organic carboxylic acid strontium salts, were measured in water. The solubility of these salts was also measured as a function of temperature. This was performed by incubating the saturated solutions of the salts in temperature controlled incubators. Furthermore the solubility of the salts was studied in pure distilled water as well as a 0.05 M ammonium carbonate buffered solutions, with a physiological pH of 7.5.

The buffered solutions were immersed into a bath of water temperature controlled at either room temperature (22 - 24 °C), at 30 °C or at 40 °C. The test tubes were stirred and the solutions were subsequently incubated in an incubater with constant temperature for 24 hours. In order to eliminate any reminiscent strontium chloride influence on the determination of solubility, all the precipitate was collected at the bottom of the test tubes and the solutions above the precipitate were carefully removed and substituted by fresh solutions. After substitution of the solutions, the test tubes were stirred again and allowed to rest for another 24 hours. From these solutions, the dissolved proportions of the strontium salt were collected in volumes of 1 mL at the specified temperature. The solutions were diluted to 50 mL before analysis by Flame Atomic Absorption Spectrometry (F-AAS). Before subsequent series of sampling, the solutions were equilibrated at the next temperature for 24 hours.

*Analysis of Strontium by flame atomic absorption spectrometry F-AAS and ICP-MS*

Two methods were used for quantification of strontium in solutions: Flame Atomic Absorption Spectrometry (F-AAS), and the more sensitive inductively-coupled-plasma-mass spectrometry (ICP-MS). For most investigations, the F-AAS method had sufficient sensitivity.

Prior to analysis of the synthesized organic strontium salts, the water solubility of some commercially available strontium salts were determined by the F-AAS method to verify the precision of the measurements and compare the obtained results with reference values for solubility of the salts. The following strontium salts were obtained: Sr- Oxalate (Aldrich 57,416-3) SrSO$_4$ (Aldrich 45,129-0) SrHPO$_4$ (Aldrich 48,042-2) and SrCl$_2$ (Aldrich 43,966-5). The solubilities were investigated as described above, and strontium content in the saturated solutions determined as described here below.

Some of the very soluble strontium salts were further diluted before analysis by F-AAS. The measurements were performed by using a Perkin-Elmer 2100 equipped with a hydrogen lamp for correction of the background signal. Strontium was measured at a slit with of 0.2 nm, the wavelength was 460.8 nm operated at an energy of 58 and a current of 8 mA.

Solutions with very low strontium content (i.e. from the analysis of solubility of strontium carbonate) were analyzed by the inductively couples plasma - mass spectrometry (ICP-MS) method. This analysis was performed using a Perkin Elmer Elan 5000 system equipped with a cross-flow nebulizer. The power was set at 1000 W and the Argon-gas flow was 12 Umin and 0.8 Umin of the torch and plasma gas, respectively.

The solubility determined for the commercially available strontium salts were in good agreement with the reference values. For most investigations, the F-AAS method had sufficient sensitivity. Table 4 presents solubilities of strontium chloride, phosphate, carbonate, oxalate and sulphate in water at 22°C. It is apparent that the experimentally determined values are in agreement with the reference values quoted for these salts. The major deviation between reference values and the experiment was obtained for strontium chloride where a lower solubility was obtained and for strontium carbonate where a significantly higher solubility was found. Since the solubility of strontium carbonate is very low, it was necessary to apply ICP-MS to the determination of the content of Sr in the supernatants from these experiments. Furthermore, the solubility of this salt will be dependent on the content of carbon dioxide in the ambient air, which was not controlled in the present experiment, providing one possible explanation for the discrepancies between the determined solubility and the reference value.

Table 4: Solubility of commercially available strontium salts in water at room temperature (22 - 24°) determined as described in example 3. Expected values refer to values quoted in scientific literature or reference material such as the 'Beilstein compendium'.

| Salt | Method | Measured g/L | Expected value 18°C (g/L) |
|------|--------|--------------|---------------------------|
| $SrCl_2$ | F-AAS | 240 | 538 |
| $SrHPO_3$ | F-AAS | 0.5 | - |
| $SrSO_4$ | F-AAS | 0.1 | 0.1 |
| $SrC_2O_4$ | F-AAS | 0.05 | 0.05 |
| $SrCO_3$ | ICP-MS | 0.00009 | 0.011 |

**Temperature and pH influence on organic strontium salt solubility**

For the majority of the organic strontium salts listed in table 2, temperature changes in the interval from 20 - 40 °C had only little influence on solubility (table 5). However, for strontium L-glutamate a significant influence of temperature on solubility was observed in the range between 20 °C and 40 °C. The solubility of this salt increased more than threefold in the investigated interval in contrast to most other salts. It is noted, that the solubility under physiological conditions (37 °C), is of relevance for the pharmaceutical use of the substances, and thus the surprising increase in strontium glutamate solubility at higher temperature may have great potential therapeutic implications.

The solubility of the strontium salts in an ammonium carbonate buffered solution of pH 7.5 was generally higher than the solubility determined in pure water (table 5). However, there were some notable exceptions, such as strontium maleate, which had decreased solubility in the buffered solution. Accordingly, it was found most relevant to compare the solubility of the strontium salts by comparing the values obtained in water, as shown in table 5.

*Relative solubility*

The water-solubilities of the organic strontium salts at room temperature and at 40 °C, are listed in table 5. The strontium salts of L-aspartate and of lactate had solubilities exceeding 50 g/l hampering exact determination of solubility with the employed experimental procedures.

The results correspond to the observations during the synthesis experiments where the citrate, the fumerate and the tartrate precipitated instantly when synthesized by the production procedures described in examples 1 and 2. This is indicative of a poor solubility of these strontium salts, as apparent by the lower solubility of these salts compared to the other organic strontium salts at both 22 °C and 40 °C.

The glutamate salt showed a higher solubility than the other salts, especially at a temperature of 40 °C. During the synthesis of this salt, it was necessary to add alcohol to the solution, to initiate crystal growth, indicative of relatively high water solubility. The other studied strontium salts only precipitated after evaporation of the solvent for a few days at room temperature, but addition of alcohol was not required to initiate crystal formation and precipitation.

Table 5. Relative solubility in water buffered solutions at pH 7.5 at 40°C and room temperature (22 - 24°C) of the investigated Strontium-salts, as determined by F-AAS.

| STRONTIUM SALT | SOLUBILITY AT ROOM TEMPERATURE (22 - 24°C) (mg/L) | | SOLUBILITY AT 40°C (mg/L) | |
|----------------|--------|--------|--------|--------|
| Anion | In water | pH 7.5 | In water | pH 7.5 |
| Malonate** | 1474 | 2816 | 1441 | 2127 |
| L-glutamate** | 2111 | 3022 | 7093 | 7195 |
| L-aspartate** | 4200 | | 7900 | |
| Pyruvate* | 2204 | 1946 | 1929 | 1829 |
| $\alpha$-ketogluterate** | 1316 | 2252 | 3534 | 3809 |
| Fumarate** † | 571 | 1215 | 444 | 977 |
| Maleate** | 3002 | 1680 | 2527 | 1457 |

(continued)

| STRONTIUM SALT | SOLUBILITY AT ROOM TEMPERATURE (22 - 24°C) (mg/L) | | SOLUBILITY AT 40°C (mg/L) | |
|---|---|---|---|---|
| Tartrate** † | 883 | 1831 | 1028 | 1400 |
| Ranelate**** † | 760 | 890 | 1450 | 1970 |
| Succinate** | 1137 | 926 | 1116 | 2233 |
| Citrate*** † | 107 | 388 | 147 | 430 |
| † Reference compounds<br>*) Mono-carboxylic acid<br>**) Di-carboxylic acid<br>***) Tri-carboxtylic acid<br>****) Quattro-carboxylic acid | | | | |

**Example 4**

**Preparation of strontium glutamate hexahydrate by synthesis at 100°C**

Initially, a suspension of glutamic acid (white colored) is prepared by adding 100 mL of millipore water to 14.703 g (0.1 moles) of solid L-glutamic acid (Sigma Aldrich, $C_5H_9NO_4$, MW 187.14 g/mole, CAS no. 142-47-2, lot. no. 426560/1, filling code 43003336) in a 250 mL beaker. To this suspension was added 26.571 g (0.1 moles) of solid strontium hydroxide (Sigma Aldrich, $Sr(OH)_2*8H_2O$, MW 265.71, CAS no. 1311-10-0). Then, a magnetic stirring rod was added and the stirring and heating was started to the point of boiling of the suspension. The final suspension is also white colored and the stirring is sustained by maintaining a medium rotation rate of the stirring apparatus. In order to prevent carbon dioxide from entering the solution, the beaker was covered by a covering glass.

After some minutes of boiling and stirring, the solution clarified and all the solid material dissolved. The boiling was maintained, and additional water was added when required, as to replace the water lost by boiling. After three hours of boiling, the solution was filtered while boiling on a Büchner funnel. Very small amounts of impurities were left in the filter. The filtrate was subsequently allowed to cool to room temperature, which resulted in growth of fine-powdered crystals of strontium glutamate hexahydrate. Precipitation of the final product progressed in the filtrate within an hour. The product was filtered and dried at 110 °C in an oven for ½ hour followed by drying 12 hours in a dessicator over silica orange. Before analysis by x-ray crystallography and by FAAS, the salts were ground to fine powder by a mortar.

The total yield of strontium glutamate hexahydrate was approximately 98% before recrystallisation, and the majority of impurities consisted of reminisces of the reagents and of strontium carbonate. This yield is significantly higher than the yield obtained by synthesis under conventional conditions where only 15 % was obtained (please see example B). Thus the high temperature synthesis method as disclosed in this patent provides a significant gain in yield and a reduction in synthesis time, while resulting In a strontium glutamate salt of higher purity. The product was unambiguously identified as strontium glutamate hexahydrate by x-ray crystallography and comparing the data to results of the literature.

Further improvements of the synthesis may include degassing by nitrogen or by argon of the water and of all aqueous solutions, which prevents contact to carbon dioxide that eventually may lead to formation of impurities of strontium carbonate. It follows that a person skilled in the art will easily be able to adapt the procedure to proceed under an inert gas atmosphere.

**Example 5**

**Preparation of strontium aspartate trihydrate by synthesis at 100 °C**

Initially, a suspension of aspartic acid (white colored) is prepared by adding 100 mL of millipore water to 13.311 g (0.1 moles) of solid L-aspartic acid (Fluka, $C_5H_9NO_4$, MW 133.11 g/mole, CAS no. 56-84-8, lot. no. 432866/1, filling code 52603495) in a 250 mL beaker. To this suspension was added 26.571 g (0.1 moles) of solid strontium hydroxide (Sigma Aldrich, $Sr(OH)_2*8H_2O$, MW 265.71, CAS no. 1311-10-0). Then, a magnetic stirring rod was added and the stirring and heating was started to the point of boiling of the suspension. The final suspension is also white colored and the stirring is sustained by maintaining a medium rotation rate of the stirring apparatus. In order to prevent carbon dioxide from entering the solution, the beaker was covered by a covering glass.

After some minutes of boiling and stirring, the solution clarified and all the solid material dissolved. The boiling was maintained, and additional water was added when required, as to replace the water lost by boiling. After three hours of boiling, the solution was filtered while boiling on a Büchner funnel. Very small amounts of impurities were left in the filter. The filtrate was subsequently allowed to cool to room temperature, which resulted in growth of fine-powdered crystals of strontium aspartate trihydrate. Precipitation of the final product progressed in the filtrate within an hour. The product was filtered and dried at 110 °C in an oven for ½ hour followed by drying 12 hours in a dessicator over silica orange. Before analysis by x-ray crystallography and by FAAS, the salts were ground to fine powder by a mortar.

The total yield of strontium aspartate trihydrate was approximately 98% before recrystallisation, and the majority of impurities consisted of reminisces of the reagents and of strontium carbonate. This yield is significantly higher than the yield obtained by synthesis under conventional conditions where only 14 % was obtained (please see example 2). Thus the high temperature synthesis method as disclosed in this patent provides a significant gain in yield and a reduction in synthesis time, while resulting In a strontium aspartate salt of higher purity. The product was unambiguously identified as strontium aspartate trihydrate by x-ray crystallography and comparing the data to results of the Cambridge Crystallographic Database.

Further improvements of the synthesis may include degassing by nitrogen or by argon of the water and of all aqueous solutions, which prevents contact to carbon dioxide that eventually may lead to formation of impurities of strontium carbonate. It follows that a person skilled in the art will easily be able to adapt the procedure to proceed under an inert gas atmosphere.

**Example 6**

**Preparation of strontium malonate monohydrate by synthesis at 100 °C**

Initially, a suspension of malonic acid (white colored) is prepared by adding 100 mL of millipore water to 10.406 g (0.1 moles) of solid malonic acid (Fluka, , MW 104.06 g/mole, CAS no. 141-82-2, lot. no. 449503/1, filling code 44903076) in a 250 mL beaker. To this suspension was added 26.571 g (0.1 moles) of solid strontium hydroxide (Sigma Aldrich, $Sr(OH)_2*8H_2O$, MW 265.71, CAS no. 1311-10-0). Then, a magnetic stirring rod was added and the stirring and heating was started to the point of boiling of the suspension. The final suspension is also white colored and the stirring was sustained by maintaining a medium rotation rate of the stirring apparatus. In order to prevent carbon dioxide from entering the solution, the beaker was covered by a covering glass.

After some minutes of boiling and stirring, the solution clarified and all the solid material dissolved. The boiling was maintained, and additional water was added when required, as to replace the water lost by boiling. After three hours of boiling, the solution was filtered while boiling on a Büchner funnel. Very small amounts of impurities were left in the filter. The filtrate was subsequently allowed to cool to room temperature, which resulted in growth of fine-powdered crystals of strontium malonate. Precipitation of the final product progressed rapidly during filtration and the majority of the product was found in the filter (unheated). Only in rare instants, the precipitation progressed in the filtrate. The product was filtered and dried at 110 °C in an oven for ½ hour followed by drying 12 hours in a dessicator over silica orange. Before analysis by x-ray crystallography and by FAAS, the salts were ground to fine powder by a mortar.

The total yield of strontium malonate was approximately 98% before recrystallisation, and the majority of impurities consisted of reminisces of the reagents and of strontium carbonate. The product was unambiguously identified as strontium malonate by x-ray crystallography and comparing the data to results of the Cambridge Crystallographic Database.

Further improvements of the synthesis may include degassing by nitrogen or by argon of the water and of all aqueous solutions, which prevents contact to carbon dioxide that eventually may lead to formation of impurities of strontium carbonate. It follows that a person skilled in the art will easily be able to adapt the procedure to proceed under an inert gas atmosphere.

**Example 7**

**Methods of manufacture of water soluble strontium salts of dicarboxylic acids using temperatures above 100 °C**

According to methods developed previously and described in examples 2 - 6, synthesis of strontium salts of dicarboxylic organic acids and especially strontium salts of amino acids can be difficult to produce in larger scale (i.e. > 1 kg) due to low yields and difficulties in separating the desired reaction products from contaminants. Strontium salts of carbonate are of special concern, as they will form as impurities when the reaction is occurring in atmospheric air containing normal levels of carbon dioxide. We have described in examples 4 - 6 that the total yield of the product when strontium salts of dicarboxylic acids are manufactured from the free acid form of the anion, and strontium hydroxide depends on temperature and on time of synthesis. In order for the reaction to reach completion, the mixture of the amino acid proper and strontium hydroxide needs boiling in water for three hours, allowing ample time for strontium in the reaction mixture to react with carbon dioxide in the air. In this example we disclose methods of improving the synthesis further by providing optimized reaction conditions, where temperature is increased above 100°C in a closed container, and where reaction times are significantly reduced.

The present example provides representative data from the optimization of conditions for synthesis of strontium glutamate in an autoclave system. Strontium glutamate is used as an example, but it the optimizations described in the example is also applicable for the synthesis of other strontium salts, where the exact reaction conditions can be optimized as disclosed in this example. The reaction temperatures must be maintained below the melting point or below the temperature of decomposition of the organic anion moiety of the desired strontium salt. As an example, malonic acid decomposes at 132-134 °C, and thus synthesis of strontium malonate must be performed at temperatures below 132°C.

Strontium L-glutamate was used as a model strontium compound in the optimisation experiments. The purity of the product was monitored by comparing to crystallographic data and by measuring the content of strontium. Ideally, the content of strontium is 25.7% in strontium L-glutamate hexahydrate, which is the product formed in these experiments. It follows that other soluble strontium salts may be prepared by similar methods with high yield and purity.

*Experimental*

Preparation of solutions: A suspension of glutamic acid (white coloured) is prepared by adding 100 mL of millipore water to 14.703 g (0.1 moles) of solid L-glutamic acid (Sigma Aldrich, $C_5H_9NO_4$, MW 187.14 g/mole, CAS no. 142-47-2, lot. no. 426560/1, filling code 43003336) in a 250 mL beaker. To this suspension was added 22.257 g, 26.571 g or 31.885 (0.08 moles, 0.1 moles or 0.12 moles) of solid strontium hydroxide (Sigma Aldrich, $Sr(OH)_2$*8$H_2O$, MW 265.71, CAS no. 1311-10-0).

*Optimisation experiments*

After preparation of the salts, the nine optimisation experiments were performed according to the settings of table 6.

Table 6. Parameters and main results of the optimisation procedure for synthesis of strontium glutamate. The pressure was monitored but not used in the optimisation process. The strontium content (%Sr) was measured by FAAS but not used as quality parameter. The yield (%) was applied as the quality parameter.

| Experiment no. | Autoclave temperature (°C) | Time of synthesis (min.) | Base-acid ratio | Total volume (ML) | Autoclave pressure (bar) | Yield% | %SR (AAS) |
|---|---|---|---|---|---|---|---|
| 1 | 125 | 15 | 0,8 | 50 | 1,55 | 94 | 25 |
| 2 | 124 | 30 | 1 | 75 | 1 | 112 | 22 |
| 3 | 124 | 60 | 1,2 | 100 | 1,6 | 121 | 21 |
| 4 | 127 | 15 | 0,8 | 100 | 1,2 | 118 | 22 |
| 5 | 132 | 30 | 1 | 50 | 1,55 | 120 | 25 |
| 6 | 132 | 60 | 1,2 | 75 | 1,6 | 50 | 22 |
| 7 | 134 | 15 | 0,8 | 75 | 1,65 | 108 | 24 |
| 8 | 134 | 30 | 1 | 100 | 1,65 | 76 | 14 |
| 9 | 132 | 60 | 1,2 | 50 | 1,65 | 82 | 24 |

*Procedure*

1. The calculated amount of acid was weighed and transferred to a bluecap autoclave bottle and the Millipore water was added. The bottle was closed and shaken, in order to obtain a finely grained suspension.

2 The calculated amount of strontium hydroxide octahydrate was weighed and added to the acid solution of (1) and the bottle was vigorously wortexed until all coarse lumps of material were transformed into fine-grained powder.

3 The bottle was placed in the autoclave and the temperature was set. While in the autoclave no additional stirring was carried out.

4 At t = 100° C the valve of the autoclave was closed and the timing was started.

5 During the autoclaving were monitored the actual temperature and the actual pressure.

6 After the time of autoclaving ended, the steam was let out, as soon as possible, with due respect to safety precautions.

7 At approx. 110° C the autoclave was opened and the solution was recovered. Again, the bottle was shook, as to obtain a high degree of mixing.

8 The solution was immediately filtered hot on a Büchner funnel after autoclaving, which left only traces of carbonate in the filter. The product precipitated from the solution during cooling to room temperature.

9 After precipitation, the product was filtered and dried in an oven for ½ an hour at 110° C. Then, it was dried in a dessicator over silica-gel orange. Finally, the product was ground to fine powder in a mortar.

10. The product was weighed after grinding and the total yield calculated.

*Preparation of strontium malonate according to the invention*

In order to confirm the applicability of the disclosed high temperature synthesis method for other strontium salts than strontium L-glutamate, strontium malonate was prepared. Basically the reaction conditions found for preparation of strontium L-glutamate was employed. A suspension of malonic acid (white coloured) is prepared by adding 100 mL of millipore water to 10.41 g (0.1 moles) of solid malonic acid (FLUKA 63290, MW 104.1) in a 250 mL beaker. To this suspension was added 22.257 g, 26.571 g or 31.885 (0.08 moles, 0.1 moles or 0.12 moles) of solid strontium hydroxide (Sigma Aldrich, $Sr(OH)_2$*$8H_2O$, MW 265.71, CAS no. 1311-10-0). The reaction procedure described above was follower, and the temperature was maintained below 130°C to avoid decomposition of malonic acid, while the reaction time was maintained at 15 min.

*Content of strontium (%Sr):*

A sample of 0.2 g was dissolved in 100 mL 0.1 M $HNO_3$ prepared in Millipore water. This solution was further diluted by a factor of 500 by a solution of 1 % KCl, and the content of strontium was determined by FAAS. The measurements were performed by using a Perkin-Elmer 2100 equipped with a hydrogen lamp for correction of the background signal. Strontium was measured at a slit with of 0.2 nm, the wavelength was 460.8 nm operated at an energy of 58 and a current of 8 mA.

*X-ray crystallography*

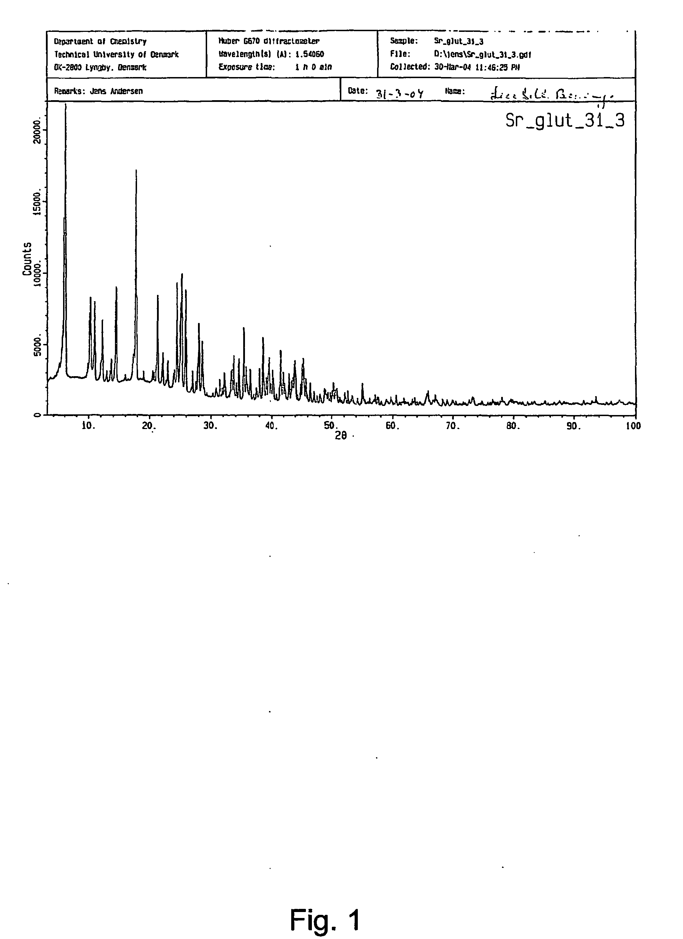

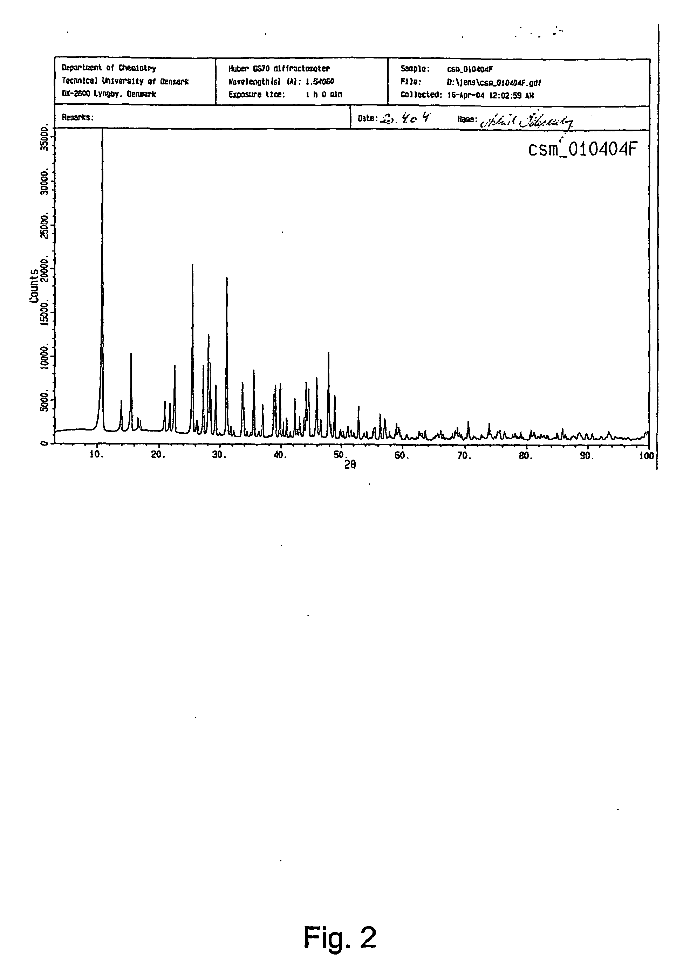

A second check of purity was performed by powder x-ray crystallography using a Huber G670 diffractometer. A characteristic diffractogram of the strontium glutamate is shown in fig. 1. An X-ray diffractogram of strontium malonate obtained by the high temperature synthesis method disclosed in the present example is shown in fig. 2. The double peak on the low angle side of the peak of maximum intensity is an artifact of the instrument.

*Results and discussion*

In table 4, it is observed that some of the synthesis conditions resulted in relatively low yield and in strontium glutamate of low purity as apparent from the molar % of strontium in the reaction product. The product of experiment no. 8 was produced in relatively low yield, and it did not contain the expected 25.7% of strontium, which was also confirmed by the x-ray analysis. Despite this outlier, in general, the outcome of the optimisation experiments is close to the expected products. Incomplete reaction provides a product of too low content of strontium while formation of strontium carbonate during the synthesis gives a too high value of the strontium content. Conditions employed in experiments 1 and 5 gave the strontium content in best agreement with the expected value. Of notice, it is also apparent although the product of experiment no. 6 was produced in low yield, it contained an amount of strontium that corresponded to the expected value.

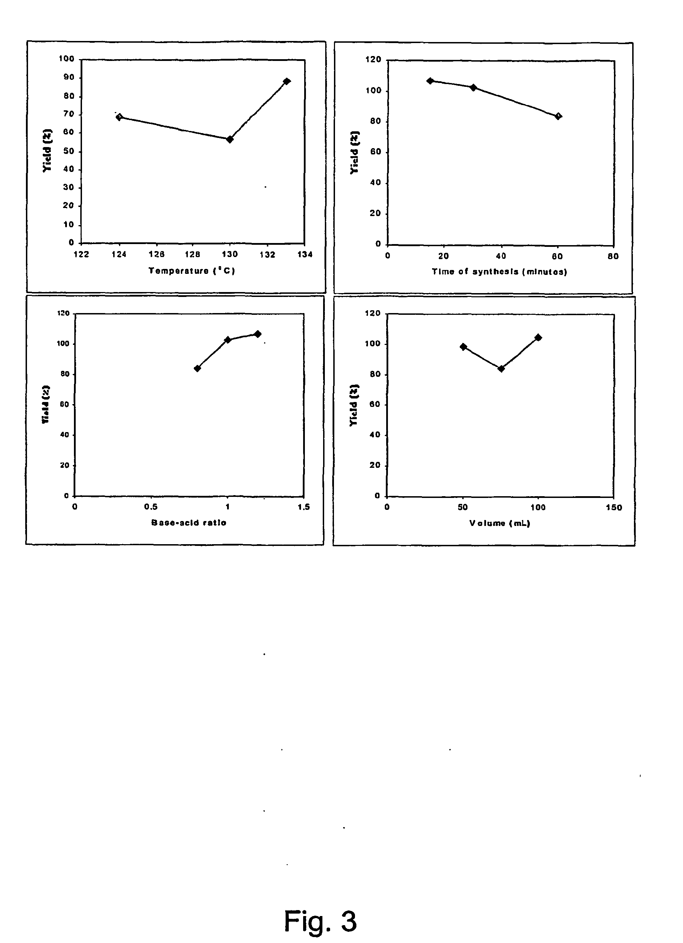

By studying the influence of the individual parameters on the total yield (table 6 and fig. 3), it becomes clear that temperature, time of autoclaving and base-acid ratio are important for the synthesis while total volume is less important. A yield higher than 100%, which is observed in experimental conditions 2,3,4,5 and 7 originates from incomplete drying, but this effect is almost eliminated when the average values are considered, as in fig. 3. Thus, the maximum yield was obtained by using a high temperature (133 °C), a short time of autoclaving (15 min.) and a surplus of strontium hydroxide. Accordingly, temperature is more important than time but it compares in importance to the base-to-acid ratio. However, great care must exerted as to not exceed the temperature of decomposition in the synthesis of other strontium salts, which for, e.g., the malonate is 132-134 °C. A 10th experiment of control of optimisation was performed, as to confirm the maximum yield of the optimisation experiments.

Furthermore, an additional experiment was performed to validate the applicability of the high temperature synthesis method for the preparation of other organic strontium salts than strontium L-glutamate. Strontium malonate was chosen, as this salt may be considered especially difficult to prepare under the high temperature conditions due to the low dissociation temperature of the malonic acid anion. However, as shown in figure 2, crystalline pure and well-defined strontium malonate could easily be obtained. The crystal structure of the compound has not been completely resolved as it is a new structure not previously described, but the data shows that the high temperature method is likely to be applicable for many other organic strontium salts.

Further improvements of the synthesis include introduction of inert atmospheres to the synthesis environment, as well as degassing of all solutions by either nitrogen gas or by argon gas, as to reduce the formation of strontium carbonate.

*Conclusion*

The optimisation experiments show that it is possible to synthesize strontium glutamate in high yields by elevating the temperature to values above 100 °C, and by using a short time (15 min.) in the autoclave. Also, a 20% surplus of strontium-hydroxide also improves the total yield without compromising the purity of the synthesized strontium salt. A slightly more vigorous drying than silica-gel orange should be applied to the drying procedure in order to obtain completely dried product. Examples of more potent drying agents are concentrated sulphuric acid or calcium oxide, but also conventional lyophilization or other mechanic treatments may be applicable for this procedure.

**Example 8** (Reference)

**Pharmacokinetic properties of organic strontium salts with low solubility**

The aim of this experiment was to assess the bioavailability of an organic strontium salt with low solubility (strontium citrate) compared with strontium chloride and strontium ranelate. The bioavailability was assessed by determination of serum strontium concentration at regular intervals over a 24 hour period and calculating AUC.

The experiment was performed with female SPF Wistar rats of the strain HanTac:WH (GALAS) from Taconic M&B A/S, Ejby, DK-4623 Lille Skensved, Denmark. At the start of the acclimatisation period, the rats were approximately 9 weeks old with a weight of approximately 200-250 g. The animals were housed in a room provided with filtered air at a temperature of 21 °C $\pm$ 3°C and relative humidity of 55% $\pm$ 15% and a ventilation system providing 10 air changes per hour. The room was illuminated to give a cycle of 12 hours light and 12 hours darkness. The rats were fed a complete pelleted rodent diet "Altromin 1314" (Chr. Petersen A/S, DK-4100 Ringsted, Denmark). The rats had free access to bottles with domestic quality drinking water acidified with hydrochloric acid to pH 2.5 in order to prevent microbial growth.

The rats were randomly allocated randomly in four groups of 9 animals treated as indicated in the table below. The groups, dose levels, animal numbers were as listed in the table 7 below:

Table 7: The 4 treatment groups of the pharmacokinetic experiment. The doses administered to each group is listed in the fist column, and salt, MW and Sr content in the middle columns.

| Dose[1] (mg /kg) | Group | Strontium salt | MW | % Sr | Dose Equivalent (Amounts in mg) | Animal No's |
|---|---|---|---|---|---|---|
| Vehicle | Control | Vehicle (0.5 % CMC) | - | - | - | 1-9 |
| 500 | B | Sr-ranelate (*7H$_2$O) | 639.6 | 27.4 | 500 = 137 mg Sr$^{++}$ | 10-18 |
| 416 | C | SrCl$_2$ (*6H$_2$O) | 266.6 | 32.9 | 137 mg Sr$^{++}$ = 416 | 19-27 |
| 390 | D | Sr-citrate (*6H$_2$O) | 749.1 | 35.1 | 137 mg Sr$^{++}$ = 390 | 28-36 |
| [1] Doses are adjusted to provide equimolar strontium dose as 500 mg/kg Strontium-ranelate (heptahydrate)(group B). | | | | | | |

The test article was given once by oral gavage according to the most recent body weight data. The control group was dosed with the vehicle alone (0.5% carboxy methyl cellulose, CMC). The vehicle was prepared with de-ionized water for all treatment groups including controls. The test substances (strontium salts) were solubilized/suspended in a volume corresponding to 5 ml/kg body weight. In order to keep the compounds in suspension, the formulations were kept on a magnetic stirrer before and during dosing.

*Blood samples for determination of strontium absorption and bioavailability*

On the day of treatment (Day 1), blood samples were taken from all animals. Blood samples were collected from 3 animals per group at the following time points: Pretreatment, and 30 min, 1, 1.5, 2, 4, 8 and 24 hours post-treatment, so that three animals from each group had samples taken at time 0, 1.5 and 6 hours, 3 other rats at time 0.5, 2, 8 hours and the remaining three animals in the group had samples taken at 1, 4 and 24 hours.

Approximately 0.5 - 0.6 ml blood was obtained at each time point from the orbital venous plexus into plain tubes for serum. The blood was kept at room temperature for 30 to 60 minutes and until centrifugation (10 min, 1270 G, +20°C). The serum was transferred to Nunc cryotubes (Nunc, Denmark) and frozen at -18°C for subsequent analysis of strontium content by graphite -furnace atomic-absorption spectrometry (GF-AAS).

*Graphite-furnace atomic-absorption spectrometry (GF-AAS)*

Concentrated HCl was added to the serum samples to a final concentration of 0.2 % HCl and the samples were then subjected to analysis using a Perkin-Elmer 2100 equipped with a hydrogen lamp for correction of the background signal. Strontium was measured at a slit with of 0.2 nm, the wavelength was 460.8 nm operated at an energy of 58 and a current of 8 mA.

*Results of the pharmacokinetic study of strontium salt absorption*

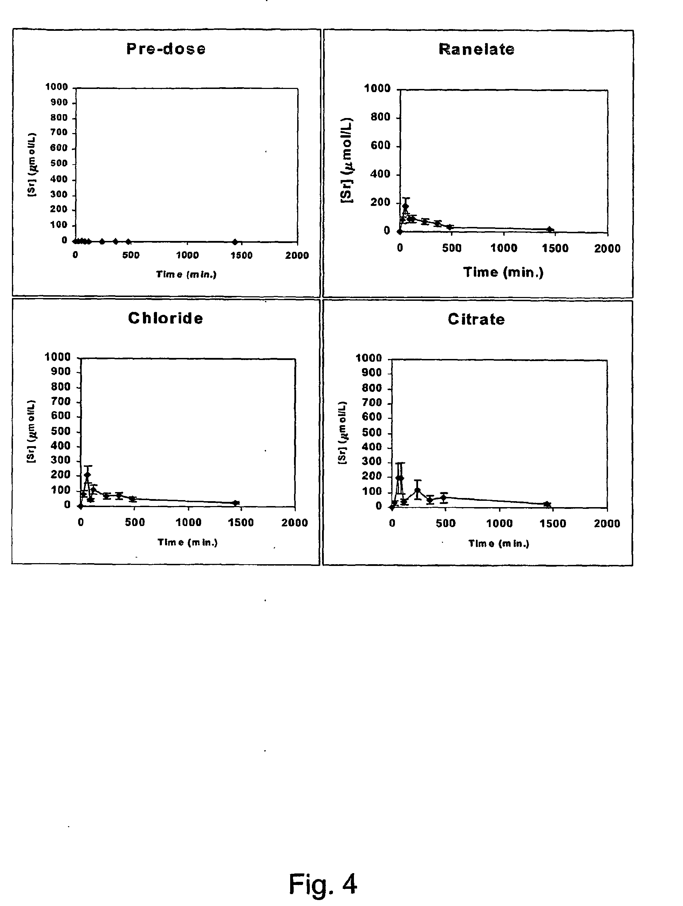

In figure 4, the serum concentration measured in the three groups treated with strontium salts are plotted as a function of the time after administration of the compounds. It is apparent that administration of the strontium salts results in a rapid and highly significant increase in serum strontium concentrations. When comparing the pharmaco-kinetic properties of different salts, it is apparent that both the highly soluble strontium chloride as well as the relatively poorly soluble strontium ranelate (see example 3), is rapidly absorbed, reaching a maximum serum concentration after approximately 2 hours. In contrast strontium citrate with the lowest solubility reaches the maximal serum concentration with a slower kinetic rate and, with maximal concentration reached after approximately 6 - 8 hours. Furthermore, the serum strontium concentration in the time interval from 0 - 8 hours after the administration of strontium citrate appears more stable.

When AUC calculations were performed the general course of the curves, as evidenced by average values in fig. 4, was best described by modelling the response/pharmacokinetic curves in a specially developed mathematical model. In the initial step, it assumes that the strontium is not metabolised but simply transferred from the stomach/upper digestive tract of the rat into epithelial cells by an active transport mechanism. Also without metabolism, the strontium ion is then transferred from the stomach/upper digestive tract where it is simultaneously released to the blood vessels. Only during the circulation of strontium through the veins, the strontium is dispersed and metabolised by the body tissue. This credible but simplified description thus includes a two-step mechanism of absorption of ionic strontium after oral administrations of strontium ions, probably corresponding to two uptake mechanisms, an active rapidly activated mechanism, and a passive transport mechanism active throughout the length of the digestive tract. After the strontium dose was administered to the rats, a characteristic time of uptake was found as t = 12 min. The maximum content of strontium in the serum was observed after approx. 30 min. The characteristic time value of 12 min. is interpreted as the duration of strontium ions being taken up by the active transport mechanism from the intestinal lumen and secreted into circulation. The time of strontium transfer between the stomach and the blood vessels is initiated almost instantly, while the time of transfer between the guts and the blood vessels proceeds at a later stage that depends on the type of salt investigated. For all salts, however, the strontium content levels out after approx. 1750 min. (29 hours) and approaches the natural level corresponding to the pre-dose level.

The model calculations were applied to the determination of the areas under the curve that are shown in table 7. The standard deviations of the AUC values correspond to the general uncertainty on the measurements of fig. 4, and their magnitude does not allow for a significant discrimination between the salts.

EP 1 622 629 B1

Table 7. Determination of the area under the curve according (AUC) to the model calculations.

| ANION OF Sr-SALT | AUC $mg / L \cdot min$ | STDDEV $mg / L \cdot min$ |
|---|---|---|
| Chloride | 7300 | 2000 |
| Citrate | 8900 | 4700 |
| vehicle | 168 | 67 |
| Ranelate | 5800 | 1700 |

These effects of delayed uptake of strontium observed with strontium citrate may enhance the pharmacologic properties of strontium. Strontium citrate resulted in the highest level of bioavailability as assessed from the AUC curve (table 7), although the differences to the other treatment groups did not reach statistical significance. The delayed attainment of $C_{max}$ may be an advantage for the use of the strontium compound in the treatment of diseases and conditions affecting bone metabolism. In these cases it is often an advantage to administer the compound in the evening before bedtime, as this would allow the compound to act at night, when resorption of bone is occurring at the highest rate. Furthermore, the administration before bedtime minimizes the potential interference from calcium in the normal diet, as the pharmaceutical preparation of the strontium salt would be taken after the last meal. This is in contrast to administration during the day, where the calcium content of normal meals would have the potential to interfere and reduce the uptake of strontium. The gradual increase in serum strontium concentration over 4 - 8 hours after administration of the compound would comply well-with evening administration of the compound and appears well suited to maximize the therapeutic effect of the strontium compound on bone metabolism.

**Claims**

1.  A tablet for medical use for oral administration once daily comprising at least one strontium salt of an organic acid, wherein the organic acid is selected from the group consisting of alpha-ketoglutaric acid, L- and D-aspartic acid, L- and D-glutamic acid, maleic acid, malonic acid [$CH_2(COOH)_2$], pyruvic acid, succinic acid [$C_2H_4(COOH)_2$] and one or more physiologically acceptable excipients, wherein the amount of strontium salt is adjusted so that the composition is for administration once daily.

2.  A tablet for use according to claim 1, wherein the water-solubility of the strontium salt is at the most 200 g/l or at the most 150 g/l, at the most 100 g/l, at the most 75 g/l, at the most 50 g/l, at the most 25 g/l, at the most 10 g/l, at the most 5 g/l, at the most 2.5 g/l, or at the most 1 g/l at room temperature (20-25 °C).

3.  A tablet for use according to claim 1 or 2, wherein the water solubility of the strontium salt is at least 0.1 g/l or in a range of from 0.1 g/l to 10 g/l, from 0.2 g/l to 5 g/l at room temperature (20-25 °C).

4.  A tablet for use according to claim 1, wherein the water solubility of the strontium salt is at least 1 g/l, or at least 5 g/l, at least 10 g/l, at least 20 g/l, at least 30 g/l, at least 40 g/l, at least 50 g/l, at least 60 g/l, at least 70 g/l, at least 80 g/l, at least 90 g/l or at least 100 g/l at room temperature of (20-25 °C).

5.  A tablet for use according to any of the preceding claims for administration once daily at bedtime

6.  A tablet for use according to any of the preceding claims comprising at least 0.01 g of strontium (calculated as ionic strontium), or at least 0.025 g, at least 0.050 g, at least 0.075 g, at least 0.1 g, at least 0.2 g, at least 0.3 g, at least 0.4 g or at least 0.5 g or from 0.01 to 2 g or from 0.1 to 2 g, from 0.1 to 1 g, from 0.15 to 0.5 g, from 0.3 to 2 g or from 0.3 to 1 g.

7.  A tablet for use according to any of claims 1-5, comprising at least 0.5 g of strontium (calculated as ionic strontium) or at least 0.6 g, at least 0.7 g least 0.8 g, at least 0.9 g, at least 1.0 g, at least 1.1 g, at least 1.2 g, at least 1.3 g, at least 1.4 g, at least 1.5 g, at least 1.6 g, at least 1.7 g, at least 1.8 g, at least 1.9 g or at least 2.0 g.

8.  A tablet for use according to any of the preceding claims in the form of a matrix tablet.

9.  A tablet for use according to claim 8 comprising 20 to 90% w/w of the active substance and 10 to 80% w/v of polymer.

22

**10.** A tablet according to any of the preceding claims, wherein the Sr salt is released from the composition in such a manner that the amplitude (difference between peak and nadir) of the plasma concentration relative to the peak level should be less than 40% or less than 35%, less than 30%, less than 25%, less than 20%, less than 15% or less than 10% after administration of the composition to a subject once daily for at least 30 days or at least 21 days, at least 14 days, at least 7 days such as 7 days.

**11.** A tablet according to any of the preceding claims, wherein the composition-when tested in an *in vitro* dissolution test - releases strontium ion from the strontium salt containing composition in the following manner:

within the first 30 min of the test at the most 10% w/w of the Sr ion is released
within the first 4 hours of the test at the most 70% w/w of the Sr ion is released
within the first 14 hours of the test 70% w/w or more of the Sr ion is released.

**12.** A tablet according to any of the preceding claims, wherein the Sr salt is contained in a matrix that governs the release.

**13.** A tablet according to any of the preceding claims, wherein the composition is coated with a controlled release coating governing the release of the Sr salt.

**14.** A tablet according to any of the preceding claims, wherein the salt is in hydrate, anhydrous, solvate, polymorphous, amorphous, crystalline, microcrystalline or polymeric form.

**15.** A tablet according to any of the preceding claims, wherein the salt is selected from the group consisting of strontium succinate, strontium malonate, strontium maleate, strontium L- and D- glutamate, strontium L- and D- aspartate, strontium pyruvate, strontium alpha-ketoglutarate and
mixtures thereof.

**16.** At least one strontium salt of an organic acid, wherein the organic acid is selected from the group consisting of alpha-ketoglutaric acid, L- and D-aspartic acid, L- and D-glutamic acid, maleic acid, malonic acid $[CH_2(COOH)_2]$, pyruvic acid and succinic acid $[C_2H_4(COOH)_2]$
and one or more physiologically acceptable excipients for medical use, to be administered in a tablet for oral administration once daily for the prophylaxis of a cartilage and/or bone condition, or for the treatment and/or prophylaxis of a cartilage and/or bone-disease and/or condition resulting in a dysregulation of cartilage and/or bone matabolism in a mammal.

**17.** At least one strontium salt for use according to claim 16 for use in preventing in a subject a cartilage and/or bone disease and/or conditions resulting in a dysregulation of cartilage and/or bone metabolism in a mammal, such as e.g. a human male or female adult, adolescent or child, such as, e.g., osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for use in the improvement of fracture healing after traumatic or atraumatic fracture, and suitable for the maintenance or increase of energy level, for building up or strengthening muscle tissues and for weight gain, wherein a tablet of one or more strontium salts of an organic acid, wherein the organic acid is selected from the group consisting of alpha-ketoglutaric acid, L- and D-aspartic acid, Land D-glutamic acid, maleic acid, malonic acid $[CH_2(COOH)_2]$, pyruvic acid, and succinic acid $[C_2H_4(COOH)_2]$ and one or more physiologically acceptable excipients is administered once daily.

**18.** At least one strontium salt for use according to claim 16 for use in treatment and/or prophylaxis of a cartilage and/or bone disease and/or conditions requiting in a dysregulation of cartilage and/or bone metabolism in a mammal, such as e.g. a human female or male adult, adolescent or child, such as e.g. osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for use in the improvement of fracture healing after traumatic or atraumatic fracture, for use in the improvement of implant stability and suitable for the maintenance or increase of energy level, for building up or strengthening muscle tissues and for weight gain, wherein the tablet comprises a single daily dose of one or more strontium salts of an organic acid, wherein the organic acid is selected from the group consisting of alpha-ketoglutaric acid, L- and D-aspartic acid, L- and D-glutamic acid, maleic acid, malonic acid [$CH_2(COOH)_2$], pyruvic acid and succinic acid [$C_2H_4(COOH)_2$], and one or more acceptable excipients comprising at least 0.5 g of strontium (calculated as strontium ion), such as, e.g, at least 0.6 g, at least about 0.7 g at least 0.8 g, at least 0.9 g, at least 1.0 g, at least 1.1 g, at least 1.2 g, at least 1.3 g, at least 1.4. g, at least 1.5 g, at least 1.6 g, at least 1.7 g, at least 1.8 g, at least 1.9 g or at least 2.0 g.

19. At least one strontium salt for use according to claim 16 for use in treatment and/or prophylaxis of a cartilage and/or bone disease and/or conditions resulting in a dysregulation of cartilage and/or bone metabolism in a male mammal, such as e.g. a human male adult, adolescent or child, such as e.g. osteoporosis, osteoarthritis, osteopetrosis, osteopenia and Paget's disease, hypercalcemia of malignancy, periodontal disease, hyperparathyroidism, periarticular erosions in rheumatoid arthritis, osteodystrophy, myositis ossificans, Bechterew's disease, malignant hypercalcemia, osteolytic lesions produced by bone metastasis, bone pain due to bone metastasis, bone loss due to sex steroid hormone deficiency, bone abnormalities due to steroid hormone treatment, bone abnormalities caused by cancer therapeutics, osteomalacia, Bechet's disease, hyperostosis, metastatic bone disease, immobilization-induced osteopenia or osteoporosis, or glucocorticoid-induced osteopenia or osteoporosis, osteoporosis pseudoglioma syndrome, idiopathic juvenile osteoporosis, for the improvement of fracture healing after traumatic or atraumatic fracture, for the improvement of implant stability and suitable for the maintenance or increase of energy level, for building up or strengthening muscle tissues and for weight gain, wherein the tablet of one or more strontium salts of an organic acid, wherein the organic acid is selected from the group consisting of alpha-ketoglutaric acid, L- and D-aspartic acid, L- and D-glutamic acid, maleic acid, malonic acid [$CH_2(COOH)_2$], pyruvic acid, and succinic acid [$C_2H_4(COOH)_2$], and one or more acceptable excipients is administered once daily in an t amount that gives a daily dose of from 0.25 g to 1.5 g free $Sr^{2+}$, or from 0.30 g to 1.5 g, from 0.40 g to 1.40 g, from 0.50 g to 1.30 g, from 0.60 g to 1.20 g, from 0.70 g to 1.10 g or from 0.80 g to 1.00 g.

20. At least one strontium salt for use according to any of claims 16-19, wherein the strontium salt is the malonate salt.

21. At least one strontium salt for use according to any of claims 16-20, wherein the tablet is for oral administration.

22. At least one strontium salt for use according to any of claims 16-21, wherein the tablets are as defined in any of claims 1-7, 9-15.

23. At least one strontium salt for use according to claims 17, wherein the subject is a female having a bone mineral density, BMD, of more than 1 SD below the young adult female mean.

24. At least one strontium salt for use according to claims 17, wherein the subject is a female having a BMD below the adult female mean for women of the same age.

25. At least one strontium salt for use according to claims 17, wherein the subject is a male having a BMD of more than 1 SD below the young adult male mean.

26. At least one strontium salt for use according to claims 17, wherein the subject is a male having a BMD below the adult male mean for men of the same age.

27. At least one strontium salt for use according to claims 17, wherein the subject is a female having a level of a specific biomarker of bone resorption, of more than 1 SD above the young adult female mean.

28. At least one strontium salt for use according to claims 17, wherein the subject is a female having a level of a specific biomarker of bone resorption above the adult female mean for women of the same age.

29. At least one strontiums salt for use according to claims 17, wherein the subject is a male having a level of a specific biomarker of bone resorption, of more than 1 SD above the young adult male mean.

30. At least one strontium salt for use according to claims 17, wherein the subject is a male having a level of a specific biomarker of bone resorption above the adult mean for men of the same age.

31. At least one strontium salt for use according to claims 17,23,24 and 27-28 for the treatment of a female of 20 years or older or 25 years or older, 30 years or older, 35 years or older, 40 years or older, 45 years or older, or 50 years or older.

32. At least one strontium salt for use according to any of claims 17, 23, 24, 27-28, 31, foruse in treatment of a female that is about the same age as her age of onset of menopause.

33. At least one strontium salt for use according to any of claims 17,23,24, 27-28, 31, wherein the subject is a female who is about 6 months or more beyond the onset of menopause.

34. At least one strontium salt for use according to any of claims 17 25-26, 29-30, for the treatment of a male of 20 years or older or 25 years or older, 30 years or older, 35 years or older, 40 years or older, 45 years or older, 50 years or older, 55 years or older, 60 years or older, 65 years or older, or 70 years or older.

35. At least one strontium salt for use according to any of claims 17, and 23-34 wherein the daily dose of strontium administered is at least 0.01 g of strontium, or at least 0.025 g, at least 0.050 g, at least 0.075 g, at least 0.1 g, at least 0.2 g, at least 0.3 g, at least 0.4 g or at least 0.5 g or from 0.01 to 2 g or from 0.1 to 2 g, from 0.1 to 1 g, from 0.15 to 0.5 g, from 0.3 to 2 g or from 0.3 to 1 g.

36. At least one strontium salt for use according to claim 16 in treating and/or preventing secondary osteoporosis in a subject, wherein an effective amount of one or more strontium salts of an organic acid, wherein the organic acid is selected from the group consisting of alpha-ketoglutaric acid, L- and D-aspartic acid, L- and D-glutamic acid, maleic acid, malonic acid $[CH_2(COOH)_2]$, pyruvic acid, and succinic acid $[C_2H_4(COOH)_2]$
and one or more physiologically acceptable excipients is administered once daily to the subject.

37. At least one strontium salt for use according to claim 36, wherein the secondary osteoporosis is induced by e.g. endocrine diseases, metabolic causes, nutritional conditions, drug substances and/or disorders of the collagen metabolism.

38. At least one strontium salt for use according to claim 16 for use in preventing drug induced secondary osteoporosis in a subject, comprising administering to the subject a prophylactic amount of one or more strontium salts of an organic acid, wherein the organic acid is selected from the group consisting of alpha-ketoglutaric acid, L- and D-aspartic acid, L- and D-glutamic acid, maleic acid, malonic acid $[CH_2(COOH)_2]$, pyruvic acid, and succinic acid $[C_2H_4(COOH)_2]$ and one or more physiologically acceptable excipients before, during or after treatment of the subject with the drug substance that induces secondary osteoporosis.

**Patentansprüche**

1. Tablette zur medizinischen Verwendung für die orale Verabreichung einmal täglich umfassend mindestens ein Strontiumsalz einer organischen Säure, wobei die organische Säure ausgewählt ist aus der Gruppe bestehend aus alpha-Ketoglutarsäure, L- und D-Asparaginsäure, L- und D-Glutaminsäure, Maleinsäure, Malonsäure $[CH_2(COOH)_2]$, Brenztraubensäure, Bernsteinsäure $[C_2H_4(COOH)_2]$ und einem oder mehreren physiologisch akzeptablen Hilfsstoffen, wobei die Menge an Strontiumsalz so eingestellt ist, dass die Verabreichung einmal täglich erfolgt.

2. Tablette zur Verwendung nach Anspruch 1, wobei die Wasserlöslichkeit des Strontiumsalzes bei Raumtemperatur (20-25°C) höchstens 200 g/l oder höchstens 150 g/l, höchstens 100 g/l, höchstens 75 g/l, höchstens 50 g/l, höchstens 25 g/l, höchstens 10 g/l höchstens 5 g/l, höchstens 2,5 g/l, oder höchstens 1 g/l beträgt.

3. Tablette zur Verwendung nach Anspruch 1 oder 2, wobei die Wasserlöslichkeit des Strontiumsalzes bei Raumtemperatur (20-25°C) mindestens 0,1 g/l ist, oder in einem Bereich von 0,1 g/l bis 10 g/l, von 0,2 g/l bis 5 g/l liegt.

4. Tablette zur Verwendung nach Anspruch 1, wobei die Wasserlöslichkeit des Strontiumsalzes bei Raumtemperatur (20-25°C) mindestens 1 g/l, oder mindestens 5 g/l, mindestens 10 g/l, mindestens 20 g/l, mindestens 30 g/l, mindestens 40 g/l, mindestens 50 g/l, mindestens 60 g/l, mindestens 70 g/l, mindestens 80 g/l, mindestens 90 g/l oder mindestens 100 g/l ist.

5. Tablette zur Verwendung nach einem der voranstehenden Ansprüche zur Verabreichung einmal täglich zur Schlafenszeit.

6. Tablette zur Verwendung nach einem der voranstehenden Ansprüche, umfassend mindestens 0,01 g Strontium (berechnet als ionisches Strontium), oder mindestens 0,025 g, mindestens 0,050 g, mindestens 0,075 g, mindestens 0,1 g, mindestens 0,2 g, mindestens 0,3 g, mindestens 0,4 g oder mindestens 0,5 g oder von 0,01 bis 2 g oder von 0,1 bis 2 g, von 0,1 bis 1 g, von 0,15 bis 0,5 g, von 0,3 bis 2 g oder von 0,3 bis 1 g.

7. Tablette zur Verwendung nach einem der Ansprüche 1 bis 5, umfassend mindestens 0,5 g Strontium (berechnet als ionisches Strontium), oder mindestens 0,6 g, mindestens 0,7 g, mindestens 0,8 g, mindestens 0,9 g, mindestens 1,0 g, mindestens 1,1 g, mindestens 1,2 g, mindestens 1,3 g, mindestens 1,4 g, mindestens 1,5 g, mindestens 1,6 g, mindestens 1,7 g, mindestens 1,8 g, mindestens 1,9 g oder mindestens 2,0 g.

8. Tablette zur Verwendung nach einem der voranstehenden Ansprüche in Form einer Matrixtablette.

9. Tablette zur Verwendung nach Anspruch 8, umfassend 20 bis 90% w/w der aktiven Substanz und 10 bis 80% w/w an Polymer.

10. Tablette zur Verwendung nach einem der voranstehenden Ansprüche, wobei das Sr-Salz aus der Zusammensetzung auf solche Weise freigesetzt wird, dass die Amplitude (Differenz zwischen Peak und Nadir) der Plasmakonzentration relativ zur Peakhöhe weniger als 40% oder weniger als 35%, weniger als 30%, weniger als 25%, weniger als 20%, weniger als 15% oder weniger als 10% nach Verabreichung der Zusammensetzung an ein Subjekt einmal täglich für mindestens 30 Tage oder mindestens 21 Tage, mindestens 14 Tage, mindestens 7 Tage, wie zum Beispiel 7 Tage sein sollte.

11. Tablette zur Verwendung nach einem der voranstehenden Ansprüche, wobei die Zusammensetzung - wenn in einem *in vitro* Auflösungstest getestet - Strontiumionen aus der Strontiumsalz enthaltenden Zusammensetzung in der folgenden Weise freisetzt:

Innerhalb der ersten 30 min des Tests werden höchstens 10% w/w des Sr-Ions freigesetzt
Innerhalb der ersten 4 Stunden des Tests werden höchstens 70% w/w des Sr-Ions freigesetzt
Innerhalb der ersten 14 Stunden des Tests werden 70% w/w oder mehr des Sr-Ions freigesetzt.

12. Tablette zur Verwendung nach einem der voranstehenden Ansprüche, wobei das Sr-Salz in einer Matrix enthalten ist, welche die Freisetzung steuert.

13. Tablette zur Verwendung nach einem der voranstehenden Ansprüche, wobei die Zusammensetzung mit einer Beschichtung zur kontrollierten Freisetzung beschichtet ist, welche die Freisetzung des Sr-Salzes steuert.

14. Tablette zur Verwendung nach einem der voranstehenden Ansprüche, wobei das Salz in Hydrat-, wasserfreier, Solvat-, polymorpher, amorpher, kristalliner, mikrokristalliner oder polymerer Form vorliegt.

15. Tablette zur Verwendung nach einem der voranstehenden Ansprüche, wobei das Salz ausgewählt ist aus der Gruppe bestehend aus Strontiumsuccinat, Strontiummalonat, Strontiummaleat, Strontium L- und D-Glutamat, Strontium L- und D-Aspartat, Strontiumpyruvat, Strontium alpha-Ketoglutarat und Gemischen davon.

16. Mindestens ein Strontiumsalz einer organischen Säure, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H4(COOH)_2$] und einem oder mehreren physiologisch akzeptablen Hilfsstoffen zur medizinischen Verwendung, die als eine Tablette zur oralen Verabreichung einmal täglich verabreicht wird, für die Prophylaxe eines Zustandes des Knorpels und/oder Knochens oder für die Behandlung und/oder die Prophylaxe einer Erkrankung des Knorpels und/oder Knochens und/oder eines Zustandes, der zu einer Dysregulierung des Stoffwechsels des Knorpels und/oder Knochens in einem Säugetier führt.

17. Mindestens ein Strontiumsalz zur Verwendung nach Anspruch 16 zur Verwendung in der Verhinderung in einem Subjekt einer Erkrankung des Knorpels und/oder Knochens und/oder von Zuständen, die zu einer Dysregulierung des Stoffwechsels des Knorpels und/oder Knochens in einem Säugetier führen, wie zum Beispiel einem männlichen oder weiblichen menschlichen Erwachsenen, Heranwachsenden oder Kind, wie zum Beispiel Osteoporose, Osteoarthritis, Osteopetrose, Osteopenie und Paget's Erkrankung, Hypercalcämie von Malignität, Periodontale Erkrankung, Hyperparathyroidismus, periartikuläre Erosionen in rheumatoider Arthritis, Osteodystrophie, Myositis Ossificans, Morbus Bechterew, maligne Hypercalcämie, osteolytische Läsionen verursacht durch Knochenmetastasen, Knochenschmerzen aufgrund von Knochenmetastasen, Knochenverlust aufgrund von Sexualsteroidhormon-Defizienz, Knochenabnormalitäten aufgrund von Steroidhormonbehandlung, Knochenabnormalitäten verursacht durch Krebstherapeutika, Osteomalazie, Morbus Bechet, Hyperostose, metastatische Knochenerkrankung, Immobilisierungs-induzierte Osteopenie oder Osteoporose oder Glukocorticoid-induzierte Osteopenie oder Osteoporose, Osteoporose Pseudogliom-Syndrom, idiopathische juvenile Osteoporose, zur Verwendung in der Verbesserung der Heilung einer Fraktur nach traumatischer oder atraumatischer Fraktur, und geeignet zur Aufrechterhaltung oder Erhöhung des Energiespiegels, zum Aufbau oder der Stärkung von Muskelgeweben und für die Gewichtszunahme, wobei eine Tablette von einem oder mehreren Strontiumsalzen einer organischen Säure, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H_4(COOH)_2$], und einem oder mehrere physiologisch akzeptablen Hilfsstoffen einmal täglich verabreicht wird.

18. Mindestens ein Strontiumsalz zur Verwendung nach Anspruch 16 zur Verwendung in der Behandlung und/oder der Prophylaxe einer Erkrankung des Knorpels und/oder Knochens und/oder von Zuständen, die zu einer Dysregulierung des Stoffwechsels des Knorpels und/oder Knochens in einem Säugetier führen, wie zum Beispiel einem männlichen oder weiblichen menschlichen Erwachsenen, Heranwachsenden oder Kind, wie zum Beispiel Osteoporose, Osteoarthritis, Osteopetrose, Osteopenie und Paget's Erkrankung, Hypercalcämie von Malignität, Periodontale Erkrankung, Hyperparathyroidismus, periartikuläre Erosionen in rheumatoider Arthritis, Osteodystrophie, Myositis Ossificans, Morbus Bechterew, maligne Hypercalcämie, osteolytische Läsionen verursacht durch Knochenmetastasen, Knochenschmerzen aufgrund von Knochenmetastasen, Knochenverlust aufgrund von Sexualsteroidhormon-Defizienz, Knochenabnormalitäten aufgrund von Steroidhormonbehandlung, Knochenabnormalitäten verursacht durch Krebstherapeutika, Osteomalazie, Morbus Bechet, Hyperostose, metastatische Knochenerkrankung, Immobilisierungs-induzierte Osteopenie oder Osteoporose oder Glukocorticoid-induzierte Osteopenie oder Osteoporose, Osteoporose Pseudogliom-Syndrom, idiopathische juvenile Osteoporose, zur Verwendung in der Verbesserung der Heilung einer Fraktur nach traumatischer oder atraumatischer Fraktur, zur Verwendung in der Verbesserung der Implantatstabilität und zur Aufrechterhaltung oder Erhöhung des Energiespiegels, zum Aufbau oder der Stärkung von Muskelgeweben und für die Gewichtszunahme, wobei die Tablette eine einzelne tägliche Dosis von einem oder mehreren Strontiumsalzen einer organischen Säure umfasst, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H_4(COOH)_2$], und einem oder mehrere physiologisch akzeptablen Hilfsstoffen umfassend mindestens 0,5 g an Strontium (berechnet als ionisches Strontium), wie zum Beispiel mindestens 0,6 g, mindestens 0,7 g, mindestens 0,8 g, mindestens 0,9 g, mindestens 1,0 g, mindestens 1,1 g, mindestens 1,2 g, mindestens 1,3 g, mindestens 1,4 g, mindestens 1,5 g, mindestens 1,6 g, mindestens 1,7 g, mindestens 1,8 g, mindestens 1,9 g oder mindestens 2,0 g.

19. Mindestens ein Strontiumsalz zur Verwendung nach Anspruch 16 zur Verwendung in der Behandlung und/oder der Prophylaxe einer Erkrankung des Knorpels und/oder Knochens und/oder von Zuständen, die zu einer Dysregulierung des Stoffwechsels des Knorpels und/oder Knochens in einem Säugetier führen, wie zum Beispiel einem männlichen oder weiblichen menschlichen Erwachsenen, Heranwachsenden oder Kind, wie zum Beispiel Osteoporose, Osteoarthritis, Osteopetrose, Osteopenie und Paget's Erkrankung, Hypercalcämie von Malignität, Periodontale Erkrankung, Hyperparathyroidismus, periartikuläre Erosionen in rheumatoider Arthritis, Osteodystrophie, Myositis Ossificans, Morbus Bechterew, maligne Hypercalcämie, osteolytische Läsionen verursacht durch Knochenmetastasen, Knochenschmerzen aufgrund von Knochenmetastasen, Knochenverlust aufgrund von Sexualsteroidhormon-Defizienz, Knochenabnormalitäten aufgrund von Steroidhormonbehandlung, Knochenabnormalitäten verursacht durch Krebstherapeutika, Osteomalazie, Morbus Bechet, Hyperostose, metastatische Knochenerkrankung, Immobilisierungs-induzierte Osteopenie oder Osteoporose oder Glukocorticoid-induzierte Osteopenie oder Osteoporose, Osteoporose Pseudogliom-Syndrom, idiopathische juvenile Osteoporose, zur Verwendung in der Verbesserung der Heilung einer Fraktur nach traumatischer oder atraumatischer Fraktur, zur Verwendung in der Verbesserung der Implantatstabilität und zur Aufrechterhaltung oder Erhöhung des Energiespiegels, zum Aufbau oder der Stärkung von Muskelgeweben und für die Gewichtszunahme, wobei die Tablette aus einem oder mehreren Strontiumsalzen einer organischen Säure, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H_4(COOH)_2$], und einem oder mehrere physiologisch akzeptablen Hilfsstoffen einmal täglich in einer Menge verabreicht wird, die eine tägliche Dosis von 0,25 g bis 1,5 g oder von 0,30 g bis 1,5 g, von 0,40 g bis 1,40 g, von 0,50 g bis 1,30 g, von 0,60 g bis 1,20 g, von 0,70 g bis 1,10 g oder von 0,80 g bis 1,00 g an freiem $Sr^{2+}$ ergibt.

20. Mindestens ein Strontiumsalz zur Verwendung nach einem der Ansprüche 16 bis 19, wobei das Strontiumsalz das Malonatsalz ist.

21. Mindestens ein Strontiumsalz zur Verwendung nach einem der Ansprüche 16 bis 20, wobei die Tablette zur oralen Verabreichung vorgesehen ist.

22. Mindestens ein Strontiumsalz zur Verwendung nach einem der Ansprüche 16 bis 21, wobei die Tabletten wie definiert nach einem der Ansprüche 1-7 und 9-15 sind.

23. Mindestens ein Strontiumsalz zur Verwendung nach Anspruch 17, wobei das Subjekt eine Frau ist, die eine Knochenmineraldichte, BMD, von mehr als 1 SD unterhalb des Mittels von jungen erwachsenen Frauen aufweist.

24. Mindestens ein Strontiumsalz zur Verwendung nach Anspruch 17, wobei das Subjekt eine Frau ist, die eine BMD von unterhalb des Mittels von erwachsenen Frauen desselben Alters aufweist.

25. Mindestens ein Strontiumsalz zur Verwendung nach Anspruch 17, wobei das Subjekt ein Mann ist, der eine BMD von mehr als 1 SD unterhalb des Mittels von jungen erwachsenen Männern aufweist.

26. Mindestens ein Strontiumsalz zur Verwendung nach Anspruch 17, wobei das Subjekt ein Mann ist, der eine BMD von unterhalb des Mittels von erwachsenen Männern desselben Alters aufweist.

27. Mindestens ein Strontiumsalz zur Verwendung nach Anspruch 17, wobei das Subjekt eine Frau ist, die einen Spiegel eines für Knochenresorption spezifischen Biomarkers von mehr als 1 SD oberhalb des Mittels von jungen erwachsenen Frauen aufweist.

28. Mindestens ein Strontiumsalz zur Verwendung nach Anspruch 17, wobei das Subjekt eine Frau ist, die einen Spiegel eines für Knochenresorption spezifischen Biomarkers von oberhalb des Mittels von erwachsenen Frauen desselben Alters aufweist.

29. Mindestens ein Strontiumsalz zur Verwendung nach Anspruch 17, wobei das Subjekt ein Mann ist, der einen Spiegel eines für Knochenresorption spezifischen Biomarkers von mehr als 1 SD oberhalb des Mittels von jungen erwachsenen Männern aufweist.

30. Mindestens ein Strontiumsalz zur Verwendung nach Anspruch 17, wobei das Subjekt ein Mann ist, der einen Spiegel eines für Knochenresorption spezifischen Biomarkers von oberhalb des Mittels von erwachsenen Männern desselben Alters aufweist.

31. Mindestens ein Strontiumsalz zur Verwendung nach Anspruch 17, 23, 24 und 27-28 zur Behandlung einer Frau von 20 Jahren oder älter, oder 25 Jahren oder älter, 30 Jahren oder älter, 35 Jahren oder älter, 40 Jahren oder älter, 45 Jahren oder älter, oder 50 Jahren oder älter.

32. Mindestens ein Strontiumsalz zur Verwendung nach Anspruch 17, 23, 24 und 27-28, 31, zur Verwendung zur Behandlung einer Frau, die in ungefähr demselben Alter wie ihr Alter bei Einsetzen der Menopause ist.

33. Mindestens ein Strontiumsalz zur Verwendung nach Anspruch 17, 23, 24 und 27-28, 31, wobei das Subjekt eine Frau ist, die sich ungefähr 6 Monate oder mehr nach dem Einsetzen der Menopause befindet.

34. Mindestens ein Strontiumsalz zur Verwendung nach Anspruch 17, 25-26, 29-30, zur Behandlung eines Mannes von 20 Jahren oder älter, oder 25 Jahren oder älter, 30 Jahren oder älter, 35 Jahren oder älter, 40 Jahren oder älter, 45 Jahren oder älter, 50 Jahren oder älter, 55 Jahren oder älter, 60 Jahren oder älter, 65 Jahren oder älter, oder 70 Jahren oder älter.

35. Mindestens ein Strontiumsalz zur Verwendung nach Anspruch 17 und 27-34, wobei die tägliche Dosis an verabreichtem Strontium mindestens 0,01 g an Strontium, oder mindestens 0,025 g, mindestens 0,050 g, mindestens 0,075 g, mindestens 0,1 g, mindestens 0,2 g, mindestens 0,3 g, mindestens 0,4 g oder mindestens 0,5 g ist, oder von 0,01 bis 2 g oder von 0,1 bis 2 g, von 0,1 bis 1 g, von 0,15 bis 0,5 g, von 0,3 bis 2 g oder von 0,3 bis 1 g ist.

36. Mindestens ein Strontiumsalz zur Verwendung nach Anspruch 16 in der Behandlung und/oder der Vorbeugung von sekundärer Osteoporose in einem Subjekt, wobei eine effektive Menge an einem oder mehreren Strontiumsalzen einer organischen Säure, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H_4(COOH)_2$], und einem oder mehreren physiologisch akzeptablen Hilfsstoffen einmal täglich an das Subjekt verabreicht wird.

37. Mindestens ein Strontiumsalz zur Verwendung nach Anspruch 36, wobei die sekundäre Osteoporose durch z.B. endokrine Erkrankungen, Stoffwechselursachen, Ernährungszustände, Drogen und/oder Abweichungen des Kollagenstoffwechsels induziert wird.

38. Mindestens ein Strontiumsalz zur Verwendung nach Anspruch 16 zur Verwendung bei der Vorbeugung von Drogen-induzierter sekundärer Osteoporose in einem Subjekt, umfassend Verabreichen an das Subjekt einer prophylaktischen Menge von einem oder mehreren Strontiumsalzen einer organischen Säure, wobei die organische Säure ausgewählt ist aus der Gruppe bestehend aus alpha-Ketoglutarsäure, L- und D-Asparaginsäure, L- und D-Glutarsäure, Maleinsäure, Malonsäure [$CH_2(COOH)_2$], Brenztraubensäure und Bernsteinsäure [$C_2H_4(COOH)_2$], und einem oder mehrere physiologisch akzeptablen Hilfsstoffen vor, während oder nach der Behandlung des Subjekts mit der Droge, die sekundäre Osteoporose induziert.


## Revendications

1. Comprimé à usage médical pour une administration orale une fois par jour comprenant au moins un sel de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2 (COOH)_2$], de l'acide pyruvique, de l'acide succinique [$C_2H_4(COOH)_2$] et d'un ou plusieurs excipients physiologiquement acceptables, dans lequel la quantité de sel de strontium est ajustée de manière à ce que la composition soit destinée à une administration une fois par jour.

2. Comprimé pour une utilisation selon la revendication 1, dans lequel la solubilité dans l'eau du sel de strontium est d'au plus 200 g/l ou d'au plus 150g/l, d'au plus 100 g/l, d'au plus 75 g/l, d'au plus 50 g/l, d'au plus 25 g/l, d'au plus 10 g/l, d'au plus 5 g/l, d'au plus 2,5 g/l, ou d'au plus 1 g/l à température ambiante (de 20 à 25 °C).

3. Comprimé pour une utilisation selon la revendication 1 ou 2, dans lequel la solubilité dans l'eau du sel de strontium est d'au moins 0,1 g/l ou est située dans une plage de 0,1 g/l à 10 g/l, de 0,2 g/l à 5 g/l à température ambiante (de 20 à 25 °C).

4. Comprimé pour une utilisation selon la revendication 1, dans lequel la solubilité dans l'eau du sel de strontium est d'au moins 1 g/l, ou d'au moins 5 g/l, d'au moins 10 g/l, d'au moins 20 g/l, d'au moins 30 g/l, d'au moins 40 g/l, d'au moins 50 g/l, d'au moins 60 g/l, d'au moins 70 g/l, d'au moins 80 g/l, d'au moins 90 g/l ou d'au moins 100 g/l à température ambiante (de 20 à 25 °C).

5. Comprimé pour une utilisation selon l'une quelconque des revendications précédentes, pour une administration une fois par jour au coucher.

6. Comprimé pour une utilisation selon l'une quelconque des revendications précédentes, comprenant au moins 0,01 g de strontium (calculé sous forme de strontium ionique), ou au moins 0,025 g, au moins 0,050 g, au moins 0,075 g, au moins 0,1 g, au moins 0,2 g, au moins 0,3 g, au moins 0,4 g ou au moins 0,5 g ou de 0,01 à 2 g ou de 0,1 à 2 g, de 0,1 à 1 g, de 0,15 à 0,5 g, de 0,3 à 2 g ou de 0,3 à 1 g.

7. Comprimé pour une utilisation selon l'une quelconque des revendications 1 à 5, comprenant au moins 0,5 g de strontium (calculé sous forme de strontium ionique) ou au moins 0,6 g, au moins 0,7 g, au moins 0,8 g, au moins 0,9 g, au moins 1,0 g, au moins 1,1 g, au moins 1,2 g, au moins 1,3 g, au moins 1,4 g, au moins 1,5 g, au moins 1,6 g, au moins 1,7 g, au moins 1,8 g, au moins 1,9 g ou au moins 2,0 g.

8. Comprimé pour une utilisation selon l'une quelconque des revendications précédentes, sous la forme d'un comprimé matriciel.

9. Comprimé pour une utilisation selon la revendication 8, comprenant de 20 à 90 % p/p de substance active et de 10 à 80 % p/v de polymère.

10. Comprimé pour une utilisation selon l'une quelconque des revendications précédentes, dans lequel le sel de Sr est libéré de la composition de manière à ce que l'amplitude (différence entre le pic et le creux) de la concentration plasmatique par rapport au niveau maximal soit inférieure à 40 % ou inférieure à 35 %, inférieure à 30 %, inférieure à 25 %, inférieure à 20 %, inférieure à 15 % ou inférieure à 10 % après l'administration de la composition à un sujet une fois par jour pendant au moins 30 jours ou au moins 21 jours, au moins 14 jours, au moins 7 jours, par exemple pendant 7 jours.

11. Comprimé pour une utilisation selon l'une quelconque des revendications précédentes, dans lequel la composition, lorsqu'elle est testée par un test de dissolution *in vitro,* libère un ion strontium à partir de la composition contenant un sel de strontium de la manière suivante :

dans les 30 premières minutes du test, au plus 10 % p/p de l'ion Sr sont libérés
dans les 4 premières heures du test, au plus 70 % p/p de l'ion Sr sont libérés
dans les 14 premières heures du test, 70 % p/p ou plus de l'ion Sr sont libérés.

12. Comprimé pour une utilisation selon l'une quelconque des revendications précédentes, dans lequel le sel de Sr est contenu dans une matrice qui gouverne la libération.

13. Comprimé pour une utilisation selon l'une quelconque des revendications précédentes, dans lequel la composition est enrobée d'un revêtement à libération contrôlée gouvernant la libération du sel de Sr.

14. Comprimé pour une utilisation selon l'une quelconque des revendications précédentes, dans lequel le sel est sous forme hydratée, anhydre, solvatée, polymorphe, amorphe, cristalline, microcristalline ou polymère.

15. Comprimé pour une utilisation selon l'une quelconque des revendications précédentes, dans lequel le sel est choisi dans le groupe constitué du succinate de strontium, du malonate de strontium, du maléate de strontium, du L- et du D-glutamate de strontium, du L- et du D-aspartate de strontium, du pyruvate de strontium, de l'alpha-cétoglutarate de strontium et de leurs mélanges.

16. Au moins un sel de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique et de l'acide succinique [$C_2H_4(COOH)_2$] et d'un ou plusieurs excipients physiologiquement acceptables à usage médical, à administrer dans un comprimé pour une administration orale une fois par jour pour la prophylaxie d'une affection cartilagineuse et/ou osseuse, ou pour le traitement et/ou la prophylaxie d'une maladie et/ou d'une affection cartilagineuse et/ou osseuse entraînant un dérèglement du métabolisme cartilagineux et/ou osseux chez un mammifère.

17. Au moins un sel de strontium pour une utilisation selon la revendication 16, pour son utilisation dans la prévention chez un sujet d'une maladie et/ou d'affections cartilagineuses et/ou osseuses entraînant un dérèglement du métabolisme cartilagineux et/ou osseux chez un mammifère, comme par exemple un adulte, un adolescent ou un enfant humain, de sexe masculin ou féminin, comme par exemple, l'ostéoporose, l'arthrose, l'ostéopétrose, l'ostéopénie et la maladie de Paget, l'hypercalcémie d'origine tumorale, les maladies parodontales, l'hyperparathyroïdisme, les érosions péri-articulaires associées à la polyarthrite rhumatoïde, l'ostéodystrophie, la myosite ossifiante, la maladie de Bechterew, l'hypercalcémie maligne, les lésions ostéolytiques produites par une métastase osseuse, les douleurs osseuses dues à une métastase osseuse, la perte osseuse due à une déficience en hormones stéroïdes sexuelles, les anomalies osseuses dues à un traitement par des hormones stéroïdes, les anomalies osseuses causées par les thérapies anticancéreuses, l'ostéomalacie, la maladie de Behçet, l'hyperostose, les maladies osseuses métastatiques, l'ostéopénie ou l'ostéoporose induites par une immobilisation, ou l'ostéopénie ou l'ostéoporose induites par les glucocorticoïdes, le syndrome ostéoporose-pseudogliome, l'ostéoporose juvénile idiopathique, pour son utilisation dans l'amélioration de la consolidation de la fracture après une fracture traumatique ou atraumatique, et convenant au maintien ou à l'augmentation du niveau énergétique, au développement ou au renforcement des tissus musculaires et à la prise de poids, dans lequel un comprimé d'un ou plusieurs sels de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique, et de l'acide succinique [$C_2H_4(COOH)_2$] et d'un ou plusieurs excipients physiologiquement acceptables est administré une fois par jour.

18. Au moins un sel de strontium pour une utilisation selon la revendication 16, pour son utilisation dans le traitement et/ou la prophylaxie d'une maladie et/ou d'affections cartilagineuses et/ou osseuses entraînant un dérèglement du métabolisme cartilagineux et/ou osseux chez un mammifère, comme par exemple un adulte, un adolescent ou un enfant humain, de sexe masculin ou féminin, comme par exemple l'ostéoporose, l'arthrose, l'ostéopétrose, l'ostéopénie et la maladie de Paget, l'hypercalcémie d'origine tumorale, les maladies parodontales, l'hyperparathyroïdisme, les érosions péri-articulaires associées à la polyarthrite rhumatoïde, l'ostéodystrophie, la myosite ossifiante, la maladie de Bechterew, l'hypercalcémie maligne, les lésions ostéolytiques produites par une métastase osseuse, les douleurs osseuses dues à une métastase osseuse, la perte osseuse due à une déficience en hormones stéroïdes sexuelles, les anomalies osseuses dues à un traitement par des hormones stéroïdes, les anomalies osseuses causées par les thérapies anticancéreuses, l'ostéomalacie, la maladie de Behçet, l'hyperostose, les maladies osseuses métastatiques, l'ostéopénie ou l'ostéoporose induites par une immobilisation, ou l'ostéopénie ou l'ostéoporose induites par les glucocorticoïdes, le syndrome ostéoporose-pseudogliome, l'ostéoporose juvénile idiopathique, pour son utilisation dans l'amélioration de la consolidation de la fracture après une fracture traumatique ou atraumatique, pour son utilisation dans l'amélioration de la stabilité des implants et convenant au maintien ou à l'augmentation du niveau énergétique, au développement ou au renforcement des tissus musculaires et à la prise de poids, dans lequel le comprimé comprend une seule dose quotidienne d'un ou plusieurs sels de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique et de l'acide succinique [$C_2H_4(COOH)_2$], et d'un ou plusieurs excipients physiologiquement acceptables comprenant au moins 0,5 g de strontium (calculé sous forme de strontium ionique), comme par exemple au moins 0,6 g, au moins environ 0,7 g, au moins 0,8 g, au moins 0,9 g, au moins 1,0 g, au moins 1,1 g, au moins 1,2 g, au moins 1,3 g, au moins 1,4 g, au moins 1,5 g, au moins 1,6 g, au moins 1,7 g, au moins 1,8 g, au moins 1,9 g ou au moins 2,0 g.

19. Au moins un sel de strontium pour une utilisation selon la revendication 16, pour son utilisation dans le traitement et/ou la prophylaxie d'une maladie et/ou d'affections cartilagineuses et/ou osseuses entraînant un dérèglement du métabolisme cartilagineux et/ou osseux chez un mammifère mâle, comme par exemple un adulte, un adolescent ou un enfant humain de sexe masculin, comme par exemple l'ostéoporose, l'arthrose, l'ostéopétrose, l'ostéopénie et la maladie de Paget, l'hypercalcémie d'origine tumorale, les maladies parodontales, l'hyperparathyroïdisme, les érosions péri-articulaires associées à la polyarthrite rhumatoïde, l'ostéodystrophie, la myosite ossifiante, la maladie de Bechterew, l'hypercalcémie maligne, les lésions ostéolytiques produites par une métastase osseuse, les douleurs osseuses dues à une métastase osseuse, la perte osseuse due à une déficience en hormones stéroïdes sexuelles, les anomalies osseuses dues à un traitement par des hormones stéroïdes, les anomalies osseuses causées par les thérapies anticancéreuses, l'ostéomalacie, la maladie de Behçet, l'hyperostose, les maladies osseuses métastatiques, l'ostéopénie ou l'ostéoporose induites par une immobilisation, ou l'ostéopénie ou l'ostéoporose induites par les glucocorticoïdes, le syndrome ostéoporose-pseudogliome, l'ostéoporose juvénile idiopathique, pour son utilisation dans l'amélioration de la consolidation de la fracture après une fracture traumatique ou atraumatique, pour son utilisation dans l'amélioration de la stabilité des implants et convenant au maintien ou à l'augmentation du niveau énergétique, au développement ou au renforcement des tissus musculaires et à la prise de poids, dans lequel le comprimé d'un ou plusieurs sels de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique et de l'acide succinique [$C_2H_4(COOH)_2$], et d'un ou plusieurs excipients physiologiquement acceptables est administré une fois par jour en une quantité t qui correspond à une dose quotidienne de 0,25 g à 1,5 g de $Sr^{2+}$ libre, ou de 0,30 g à 1,5 g, de 0,40 g à 1,40 g, de 0,50 g à 1,30 g, de 0,60 g à 1,20 g, de 0,70 g à 1,10 g ou de 0,80 g à 1,00 g.

20. Au moins un sel de strontium pour une utilisation selon l'une quelconque des revendications 16 à 19, lequel sel de strontium est le sel de malonate.

21. Au moins un sel de strontium pour une utilisation selon l'une quelconque des revendications 16 à 20, dans lequel le comprimé est destiné à une administration orale.

22. Au moins un sel de strontium pour une utilisation selon l'une quelconque des revendications 16 à 21, dans lequel les comprimés sont tels que définis dans l'une quelconque des revendications 1 à 7, 9 à 15.

**23.** Au moins un sel de strontium pour une utilisation selon la revendication 17, dans lequel le sujet est un sujet de sexe féminin ayant une densité minérale osseuse, DMO, de plus de 1 ET inférieure à la moyenne des jeunes adultes de sexe féminin.

**24.** Au moins un sel de strontium pour une utilisation selon la revendication 17, dans lequel le sujet est un sujet de sexe féminin ayant une DMO inférieure à la moyenne des adultes de sexe féminin pour les femmes du même âge.

**25.** Au moins un sel de strontium pour une utilisation selon la revendication 17, dans lequel le sujet est un sujet de sexe masculin ayant une DMO de plus de 1 ET inférieure à la moyenne des jeunes adultes de sexe masculin.

**26.** Au moins un sel de strontium pour une utilisation selon la revendication 17, dans lequel le sujet est un sujet de sexe masculin ayant une DMO inférieure à la moyenne des adultes de sexe masculin pour les hommes du même âge.

**27.** Au moins un sel de strontium pour une utilisation selon la revendication 17, dans lequel le sujet est un sujet de sexe féminin ayant un niveau de biomarqueur spécifique de la résorption osseuse de plus de 1 ET supérieur à la moyenne des jeunes adultes de sexe féminin.

**28.** Au moins un sel de strontium pour une utilisation selon la revendication 17, dans lequel le sujet est un sujet de sexe féminin ayant un niveau de biomarqueur spécifique de la résorption osseuse supérieur à la moyenne des adultes de sexe féminin pour les femmes du même âge.

**29.** Au moins un sel de strontium pour une utilisation selon la revendication 17, dans lequel le sujet est un sujet de sexe masculin ayant un niveau de biomarqueur spécifique de la résorption osseuse de plus de 1 ET supérieur à la moyenne des jeunes adultes de sexe masculin.

**30.** Au moins un sel de strontium pour une utilisation selon la revendication 17, dans lequel le sujet est un sujet de sexe masculin ayant un niveau de biomarqueur spécifique de la résorption osseuse supérieur à la moyenne des adultes pour les hommes du même âge.

**31.** Au moins un sel de strontium pour une utilisation selon les revendications 17, 23, 24 et 27-28, pour le traitement d'un sujet de sexe féminin de 20 ans ou plus ou de 25 ans ou plus, de 30 ans ou plus, de 35 ans ou plus, de 40 ans ou plus, de 45 ans ou plus, ou de 50 ans ou plus.

**32.** Au moins un sel de strontium pour une utilisation selon l'une quelconque des revendications 17, 23, 24, 27-28, 31, pour son utilisation dans le traitement d'un sujet de sexe féminin qui est environ à l'âge de l'apparition de la ménopause.

**33.** Au moins un sel de strontium pour une utilisation selon l'une quelconque des revendications 17, 23, 24, 27-28, 31, dans lequel le sujet est un sujet de sexe féminin qui est environ 6 mois ou plus au-delà de l'apparition de la ménopause.

**34.** Au moins un sel de strontium pour une utilisation selon l'une quelconque des revendications 17, 25-26, 29-30, pour le traitement d'un sujet de sexe masculin de 20 ans ou plus ou de 25 ans ou plus, de 30 ans ou plus, de 35 ans ou plus, de 40 ans ou plus, de 45 ans ou plus, de 50 ans ou plus, de 55 ans ou plus, de 60 ans ou plus, de 65 ans ou plus, ou de 70 ans ou plus.

**35.** Au moins un sel de strontium pour une utilisation selon l'une quelconque des revendications 17 et 23-34, dans lequel la dose quotidienne de strontium administrée est d'au moins 0,01 g de strontium, ou d'au moins 0,025 g, d'au moins 0,050 g, d'au moins 0,075 g, d'au moins 0,1 g, d'au moins 0,2 g, d'au moins 0,3 g, d'au moins 0,4 g ou d'au moins 0,5 g ou de 0,01 à 2 g ou de 0,1 à 2 g, de 0,1 à 1 g, de 0,15 à 0,5 g, de 0,3 à 2 g ou de 0,3 à 1 g.

**36.** Au moins un sel de strontium pour une utilisation selon la revendication 16, dans le traitement et/ou la prévention de l'ostéoporose secondaire chez un sujet, dans lequel une quantité efficace d'un ou plusieurs sels de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique et de l'acide succinique [$C_2H_4(COOH)_2$] et d'un ou plusieurs excipients physiologiquement acceptables est administrée une fois par jour au sujet.

**37.** Au moins un sel de strontium pour une utilisation selon la revendication 36, dans lequel l'ostéoporose secondaire est induite par exemple par des maladies endocriniennes, des causes métaboliques, des affections d'origine nutritionnelle, des substances médicamenteuses et/ou des troubles du métabolisme du collagène.

**38.** Au moins un sel de strontium pour une utilisation selon la revendication 16, pour son utilisation dans la prévention de l'ostéoporose secondaire induite par des médicaments chez un sujet, comprenant l'administration au sujet d'une quantité prophylactique d'un ou plusieurs sels de strontium d'un acide organique, dans lequel l'acide organique est choisi dans le groupe constitué de l'acide alpha-cétoglutarique, de l'acide L- et D-aspartique, de l'acide L- et D-glutamique, de l'acide maléique, de l'acide malonique [$CH_2(COOH)_2$], de l'acide pyruvique et de l'acide succinique [$C_2H_4(COOH)_2$] et d'un ou plusieurs excipients physiologiquement acceptables avant, pendant ou après le traitement du sujet par la substance médicamenteuse qui induit l'ostéoporose secondaire.

EP 1 622 629 B1

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0415850 B

**Non-patent literature cited in the description**

- **SORBERA LA et al.** STRONTIUM RANELATE TREATMENT AND PREVENTION OF OSTEOPOROSIS BONE RESORPTION INHIBITOR BONE FORMATION STIMULANT. *DRUGS OF THE FUTURE, PROUS SCIENCE, ES,* 01 April 2003, vol. 28, 328-335

- **REGINSTER, JY.** *Curr pharm Des,* 2002, vol. 8 (21), 1907-16